# EUROPEAN PATENT SPECIFICATION

(11) **EP 3 801 589 B1**
(45) Date of publication and mention of the grant of the patent: **28.08.2024**
(21) Application number: 19809981.4
(22) Date of filing: 31.05.2019
(51) Int. Cl.: A61K 35/15, A61K 38/17, A61P 19/08, C12N 5/077

(54) **METHODS AND COMPOSITIONS FOR THE TREATMENT OF OSTEOPETROSIS**
VERFAHREN UND ZUSAMMENSETZUNGEN ZUR BEHANDLUNG VON OSTEOPETROSE
MÉTHODES ET COMPOSITIONS POUR LE TRAITEMENT DE L'OSTÉOPÉTROSE

(30) Priority: 01.06.2018 US 201862679553 P
(43) Date of publication of application: 14.04.2021
(73) Proprietor: Memorial Sloan Kettering Cancer Center, New York, NY 10065 (US)
(72) Inventor: GEISSMANN, Frederic, New York, New York 10065 (US); MULLER, James T., New York, New York 10065 (US)
(74) Representative: Barker Brettell LLP
(86) International application number: PCT/US2019/034984
(87) International publication number: WO 2019/232426

(56) References cited:
- US-A1- 2014 127 268
- US-A1- 2015 093 363
- US-A1- 2015 353 890
- US-B2- 10 881 611
- US-B2- 9 452 187
- WU CALVIN C ET AL: "Diagnosis and Management of Osteopetrosis: Consensus Guidelines From the Osteopetrosis Working Group", JOURNAL OF CLINICAL ENDOCRINOLOGY AND METABOLISM, vol. 102, no. 9, 1 September 2017 (2017-09-01), US, pages 3111 - 3123, XP055843693, ISSN: 0021-972X, Retrieved from the Internet <URL:https://watermark.silverchair.com/jc.2017-01127.pdf?token=AQECAHi208BE49Ooan9kkhW_Ercy7Dm3ZL_9Cf3qfKAc485ysgAAAs8wggLLBgkqhkiG9w0BBwagggK8MIICuAIBADCCArEGCSqGSIb3DQEHATAeBglghkgBZQMEAS4wEQQM-vV_0jEPwe7fSzzbAgEQgIICgkMOfU2Nti0KigcQOq31nP4SZK2BJI5VIzSaa1FTfjAq9oRc3KIEoNampb0n1KWiCUKKN7dfKxPISajvOZg1iZ> DOI: 10.1210/jc.2017-01127
- XIAN XIAOJIE ET AL: "Conclusions", STEM CELL RESEARCH & THERAPY, vol. 11, no. 1, 1 December 2020 (2020-12-01), XP055843686, Retrieved from the Internet <URL:https://stemcellres.biomedcentral.com/track/pdf/10.1186/s13287-020-01701-y.pdf> DOI: 10.1186/s13287-020-01701-y
- MOSCATELLI ILANA ET AL: "Gene therapy for infantile malignant osteopetrosis: review of pre-clinical research and proof-of-concept for phenotypic reversal", MOLECULAR THERAPY- METHODS & CLINICAL DEVELOPMENT, vol. 20, 1 March 2021 (2021-03-01), GB, pages 389 - 397, XP055843688, ISSN: 2329-0501, Retrieved from the Internet <URL:https://www.cell.com/action/showPdf?pii=S2329-0501(20)30257-6> DOI: 10.1016/j.omtm.2020.12.009
- PENNA SARA ET AL: "One Disease, Many Genes: Implications for the Treatment of Osteopetroses", FRONTIERS IN ENDOCRINOLOGY, vol. 10, 19 February 2019 (2019-02-19), XP055843691, Retrieved from the Internet <URL:https://www.ncbi.nlm.nih.gov/pmc/articles/PMC6389615/pdf/fendo-10-00085.pdf> DOI: 10.3389/fendo.2019.00085
- DOUGALL ET AL.: "RANK is essential for osteoclast and lymph node development", GENES DEV, vol. 13, 15 September 1999 (1999-09-15), pages 2412 - 2424, XP002228678, DOI: 10.1101/gad.13.18.2412
- SAFTIG ET AL.: "Impaired osteoclastic bone resorption leads to osteopetrosis in cathepsin-K-deficient mice", PROC NATL ACAD SCI USA, vol. 95, 10 November 1998 (1998-11-10), pages 13453 - 13458, XP008111281
- GUERRINI ET AL.: "Human osteoclast-poor osteopetrosis with hypogammaglobulinemia due to TNFRSF11A (RANK) mutations", AM J HUM GENET, vol. 83, 11 July 2008 (2008-07-11), pages 64 - 76, XP055658131
- "pEGFP-N1 Vector Information", PROTOCOL # PT 3027-5, 19 March 1999 (1999-03-19), pages 1 - 3, XP055658138, Retrieved from the Internet <URL:http://www.yrgene.com/documents/vector/pegfpn1.pdf> [retrieved on 20190915]
- JACOME-GALARZA ET AL.: "Developmental origin, functional maintenance and genetic rescue of osteoclasts", NATURE, vol. 568, 10 April 2019 (2019-04-10), pages 541 - 545, XP036900382, DOI: 10.1038/s41586-019-1105-7

## Description

### CROSS-REFERENCE TO RELATED APPLICATION

The present application claims priority to U.S. Provisional Patent Application No. 62/679,553, filed on June 1, 2018.

### STATEMENT OF FEDERALLY FUNDED RESEARCH

This invention was made with government support under CA008748, AI130345 and HL 13 8090 awarded by the National Institutes of Health. The government has certain rights in the invention.

### TECHNICAL FIELD

The present technology generally relates to methods and compositions for treating, preventing, or ameliorating osteopetrosis. More specifically, the present technology relates to administering a composition comprising a therapeutically effective amount of engineered monocytic cells or wild-type monocytic cells from a healthy donor to a subject suffering from or at risk for osteopetrosis.

### BACKGROUND

The following description is provided to assist the understanding of the reader. None of the information provided or references cited is admitted to be prior art.

Osteoclasts are multinucleated giant cells that resorb bone, ensuring development and continuous remodeling of the skeleton and the bone marrow hematopoietic niche. Defective osteoclast activity leads to osteopetrosis and bone marrow failure, while excess activity can contribute to bone loss and osteoporosis. Osteopetrosis can be partially treated by bone marrow transplantation in human and mice, in accordance with osteoclasts hematopoietic origin, and studies suggesting that they develop by fusion of hematopoietic stem cell (HSC)-derived monocytic precursors in the presence of CSF1 and RANK-Ligand. However, the developmental origin and lifespan of osteoclasts, and the mechanisms that ensure maintenance of osteoclast function throughout life *in vivo* remain largely unexplored. Moreover, there is a need to develop alternative therapeutic approaches to the treatment of osteopetrosis as the current treatment by bone marrow transplantation is plagued by an approximate 48% overall survival at 6 years.
US 9452187 and US 2015/353890 disclose HSC transplantation for treating osteopetrosis.

### SUMMARY

The invention is defined in the claims. In one aspect, the present disclosure provides a composition comprising an effective amount of monocytic cells that have been obtained from a healthy donor for use in treating or preventing osteopetrosis in a subject in need thereof. In some embodiments, the subject is characterized by decreased expression of one or more of *CA2*, *CLCN7*, *CTSK, CSF1R*, *IKBKG, ITGB3, OSTM1, PLEKHM1, TCIRG1, TNFRSF11A*, and *TNFSF11*, as compared to the monocytic cells of the donor. In some embodiments, the osteopetrosis comprises one or more of stunted growth, skeletal deformity, increased likelihood of bone fracture, anemia, recurrent infections, hepatosplenomegaly, facial paralysis, abnormal cortical bone morphology, abnormal form of vertebral bodies, abnormal temperature regulation, abnormality of the ribs, abnormality of vertebral epiphysis morphology, bone pain, cranial nerve paralysis, craniosynostosis, hearing impairment, and hypocalcemia. In some embodiments, the composition is formulated for intravenous administration by injection, infusion, or transfusion. In some embodiments, the subject is a mammal. In some embodiments, the mammalian subject is a human. In some embodiments, the subject is characterized by a cathepsin K deficiency. In some embodiments, the subject is diagnosed as having pycnodysostosis. In some embodiments, the subject is diagnosed as having severe infantile autosomal recessive osteopetrosis.

In one aspect, the present disclosure provides a composition comprising an effective amount monocytic cells engineered to express one or more genes selected from *CA2*, *CLCN7*, *CTSK, CSF1R, IKBKG, ITGB3, OSTM1, PLEKHM1, TCIRG1*, *TNFRSF11A*, and *TNFSF11*, for use in treating or preventing osteopetrosis in a subject in need thereof. In some embodiments, the osteopetrosis comprises one or more of stunted growth, skeletal deformity, increased likelihood of bone fracture, anemia, recurrent infections, hepatosplenomegaly, facial paralysis, abnormal cortical bone morphology, abnormal form of vertebral bodies, abnormal temperature regulation, abnormality of the ribs, abnormality of vertebral epiphysis morphology, bone pain, cranial nerve paralysis, craniosynostosis, hearing impairment, and hypocalcemia. In some embodiments, the composition is formulated for intravenous administration by injection, infusion, or transfusion. In some embodiments, the subject is a mammal. In some embodiments, the mammalian subject is a human. In some embodiments, the subject is characterized by a cathepsin K deficiency. In some embodiments, the monocytic cells are obtained from the subject. In some embodiments, the subject is diagnosed as having pycnodysostosis. In some embodiments, the subject is diagnosed as having severe infantile autosomal recessive osteopetrosis.

In one aspect, the present disclosure provides a donor monocytic cell line engineered to express one or more genes selected from *CA2*, *CLCN7*, *CTSK*, *CSF1R*, *IKBKG, ITGB3, OSTM1, PLEKHM1, TCIRG1, TNFRSF11A,* and *TNFSF11,* wherein the one or more genes is operably linked to a heterologous nucleic acid to form a chimeric nucleic acid construct. In some embodiments, the heterologous nucleic acid encodes a selectable marker. In some embodiments, the selectable marker is a bioluminescent protein, a fluorescent protein, a chemiluminescent protein, a xanthine-guanine phosphoribosyl transferase gene (gpt), or any combination thereof. In some embodiments, the heterologous nucleic acid encodes one or more control sequences suitable for directing expression of the one or more genes in a monocytic cell. In some embodiments, the one or more control sequences comprises a promoter. In some embodiments, the donor cells comprise a vector encoding one or more genes selected from *CA2, CLCN7, CTSK, CSF1R, IKBKG, ITGB3, OSTM1, PLEKHM1, TCIRG1, TNFRSF11A,* and *TNFSF11.* In some embodiments, the vector is a mammalian expression vector, a lentiviral vector, or transposon vector.

The invention is defined by the claims. Any references in the description to methods of treatment refer to the compositions of the present invention in a method for treatment.

### BRIEF DESCRIPTION OF THE DRAWINGS

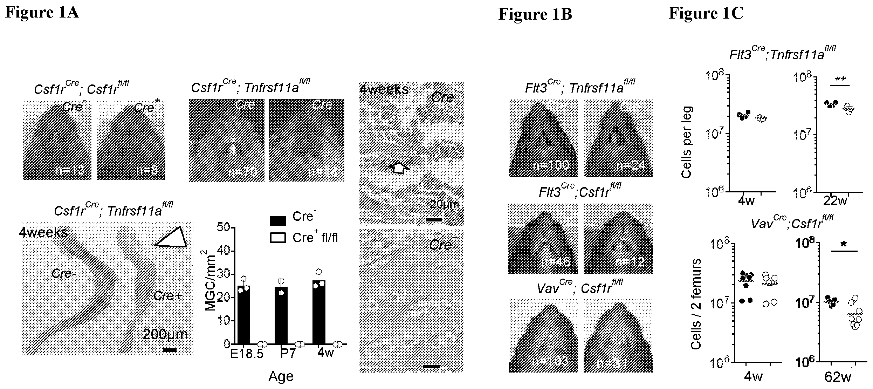

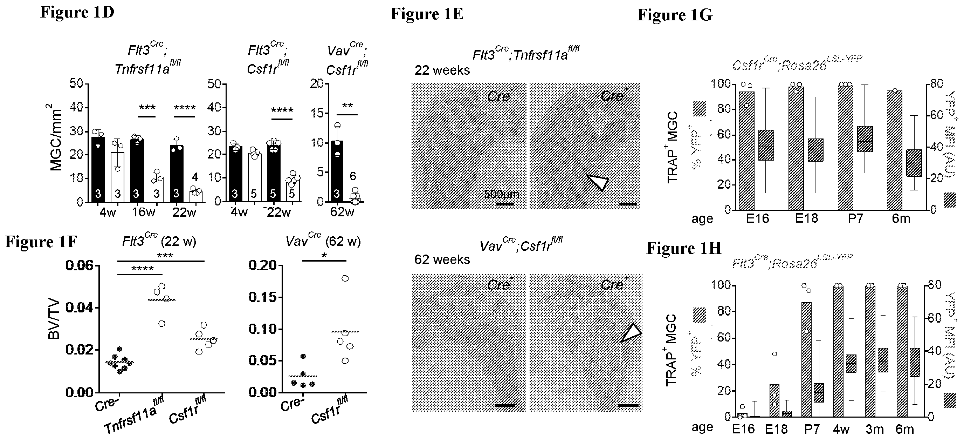

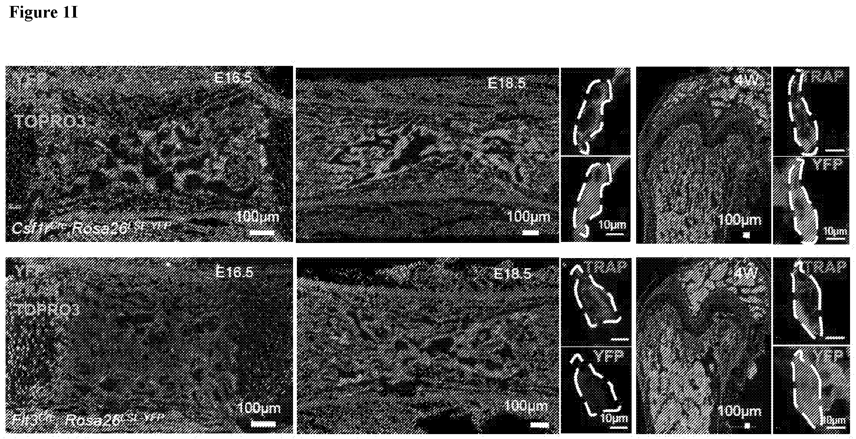

**Figures 1A-1I****.** *HSC-derived precursors are dispensable for osteoclasts and bone development.* **Figure 1A****:** Top: Representative photographs of teeth of control littermates and *Csf1r^{Cre}; Csf1r*^{*fl*/*fl*} 3-4 week old mice (right) and controls and *Csf1r^{Cre}; Tnfrsf11a*^{*fl*/*fl*} mice (left). Bottom left: Representative photographs of leg bones from controls and *Csf1r^{Cre}; Tnfrsf11a*^{*fl*/*fl*} mice, white arrowhead points to lack of blood cells. Right: bar graphs represent osteoclast numbers in E18.5, P7, and 4-week old *Csf1r^{Cre}; Tnfrsf11a*^{*fl*/*fl*} mice and littermates (n=3/3), and representative hematoxylin/TRAP staining of femur sections; arrow indicates an osteoclast. **Figure 1B****:** Representative photographs of teeth of 3- 4 week old *Flt3^{Cre}; Tnfrsf11a*^{*fl*/*fl*}, *Flt3^{Cre};Csf1r*^{*fl*/}*^{fl}, Vav^{Cre};Csf1r*^{*fl*/*fl*} and littermates. **Figure 1C****:** Bone marrow CD45+ cells numbers in *Flt3^{Cre};Tnfrsf11a*^{*fl*/*fl*} and littermates at 4 (n=4/3) and 22 week old (n=4/5) and *Vav^{Cre};Csf1r*^{*fl*/*fl*} and littermates at 4 (n=8/8) and 62 week old (n=5/8) of the indicated age. **Figure 1D****:** Osteoclast counts in femurs from *Flt3^{Cre}; Tnfrsf11a*^{*fl*/*f*l}*, Flt3^{Cre};Csf1r*^{*fl*/*fl*}, *Vav^{Cre};Csf1r*^{*fl*/*fl*} mice and littermates of the indicated ages. **Figure 1E****:** Representative hematoxylin/TRAP staining of femur sections from 22 week old *Flt3^{Cre}; Tnfrsf11a*^{*fl*/}*^{fl},* 62 week old *Vav*^{*DD*/}*^{Cre};Csf1r*^{*fl*/*fl*} mice and littermates. White arrowhead points to trabecular bone. **Figure 1F****:** Quantitative analysis of bone volume/total volume ratio of humerus or femurs from *Flt3^{Cre};Tnfrsf11a*^{*fl*/*fl*} (n=4), *Flt3^{Cre};Csf1r*^{*fl*/*fl*} (n=5) and controls littermates (n=8), and *Vav^{Cre};Csf1r*^{*fl*/*fl*} (n=5) mice and controls littermates (n=5) as determined by MicroCT and Statistical significance determined by ANOVA. **Figure 1G****:** Expression and MFI of YFP in in TRAP⁺ multinucleated cells from *Csf1r^{Cre};Rosa26^{LSL-YFP}* mice at embryonic day E16.5, E18.5, P7, and 6 months, (n=3 per time point). For MFI and percentages, at least 100 osteoclasts were quantified per time point, genotype, displayed as a box plot, min, Q1, median, Q3 and max. **Figure 1H** as in Figure 1G for *Flt3^{Cre};Rosa26^{LSL-YFP}* mice at E16.5, E18.5, P7, 4 weeks (4w), 3 months, and 6 months. Data are mean ± SD, circles represent individual independent biological replicates, n indicates number of mice per group. Statistical significance was analysed with GraphPad Prism using unpaired two-tailed t-tests unless otherwise indicated. **Figure 1I****:** Representative confocal microscopy image of frozen sections from mice in (Figures 1G, 1H). Statistical significance was analysed with GraphPad Prism using unpaired t-tests and two-way ANOVA with Tukey's multiple comparisons test as indicated in the figure legends. Significance was considered at p value (p) *p < 0.05; **p < 0.01; ***p < 0.001; ****p < 0.0001.

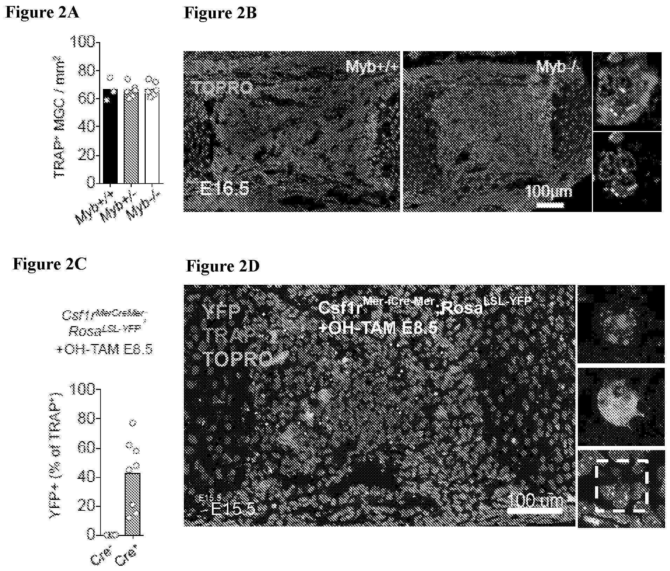

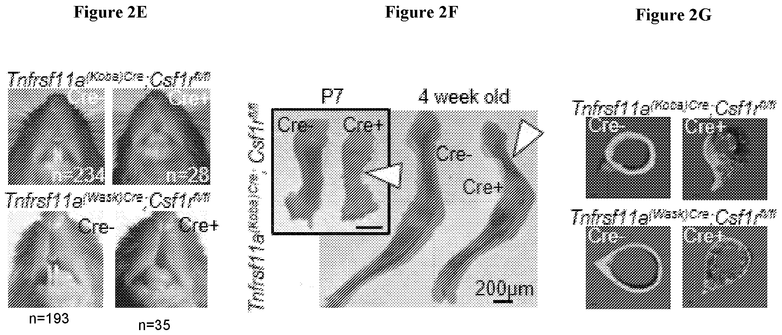

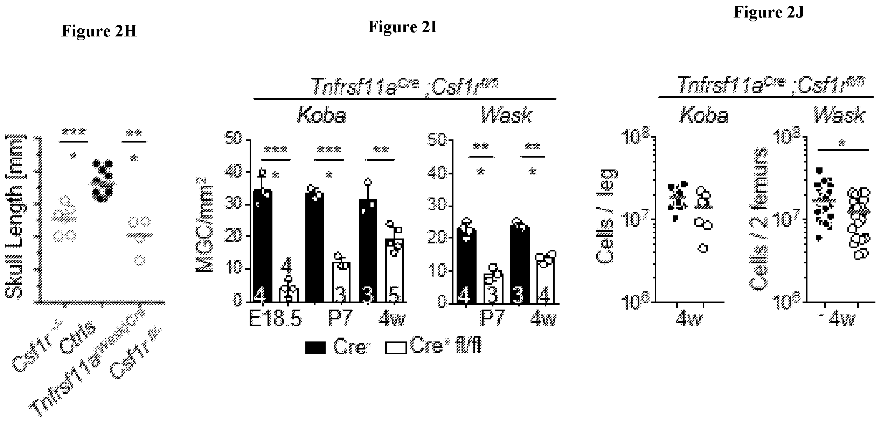

**Figures 2A-2J****.** *EMP derived osteoclasts are required for bone development.* **Figure 2A****:** MGC number in femur *anlage's* ossification centers from E15.5 / E16.5 *Myb*^{*-*/*-*} (n=6) and littermate controls (n=3). **Figure 2B****:** Representative confocal microscopy pictures of frozen sections from Figure 1A stained with fluorescent TRAP and TO-PRO-3. **Figure 2C****:** Percent of TRAP+ cells expressing YFP in femur *anlage* from E15.5 *Csf1r^{MerCreMer};Rosa26^{LSL-YFP}* mice (n=8) and Cre negative controls (n=5), pulsed at E8.5 with 4-OHT. **Figure 2D****:** Representative confocal microscopy picture from Figure 2C. **Figure 2E****:** Representative photographs of teeth of control littermates (Cre⁻), *Tnfrsf11a^{Koba-Cre};Csf1r*^{*fl*/}*^{fl} and Tnfrsf11a^{Wask-Cre};Csf1r*^{*fl*/*fl*} mice (Cre⁺). **Figure 2F****:** Leg bones from *Trfrsf11a^{Cre};Csf1r*^{*fl*/*fl*} mice (Cre⁺, n=6) and control littermates (Cre⁻, n=6) at P7and 4 weeks. Arrowhead points to the color of bone. **Figure 2G****:** Representative MicroCT scans of long bones from mice in Figure 2E (n=6 per genotype). **Figure 2H****:** Skull length from 3 weeks old *Csf1r*^{*-*/*-*} (n=6) controls littermates(n=12) and *Tnfrsf11a^{Wask-Cre};Csf1r*^{*fl*/*fl*} mice (n=4) as determined by MicroCT. **Figure 21:** Osteoclasts counts in bone sections from E18.5, P7, and 3-4 week old *Tnfrsf11a^{Cre};Csf1r*^{*fl*/*fl*} mice and littermate controls. **Figure 2J****:** Numbers of bone marrow CD45+ cells determined by flow cytometry in 4 weeks old littermates (Cre⁻, n=13,27), *Tnfrsf11a^{Koba-Cre};Csf1r*^{*fl*/*fl*} (n=9) *and Tnfrsf11a^{Wask-Cre};Csf1r*^{*fl*/*fl*} (n=23) mice (Cre⁺). Data are mean ± SD, circles on graphs represent individual mice, n indicates the number of mice per group. Statistical significance was analysed with GraphPad Prism using unpaired two tailed t-tests. Significance was considered at p value (p) *p < 0.05; **p < 0.01; ***p < 0.001; ****p < 0.0001.

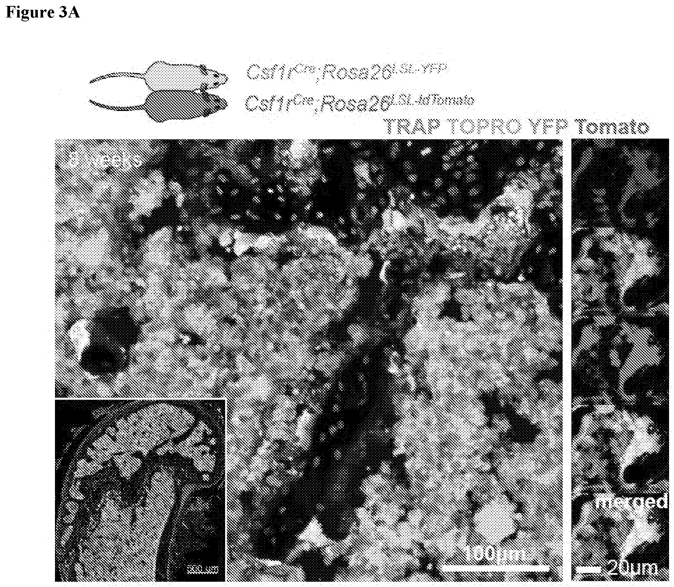

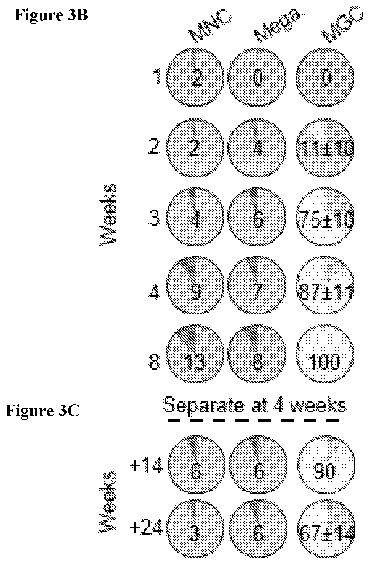

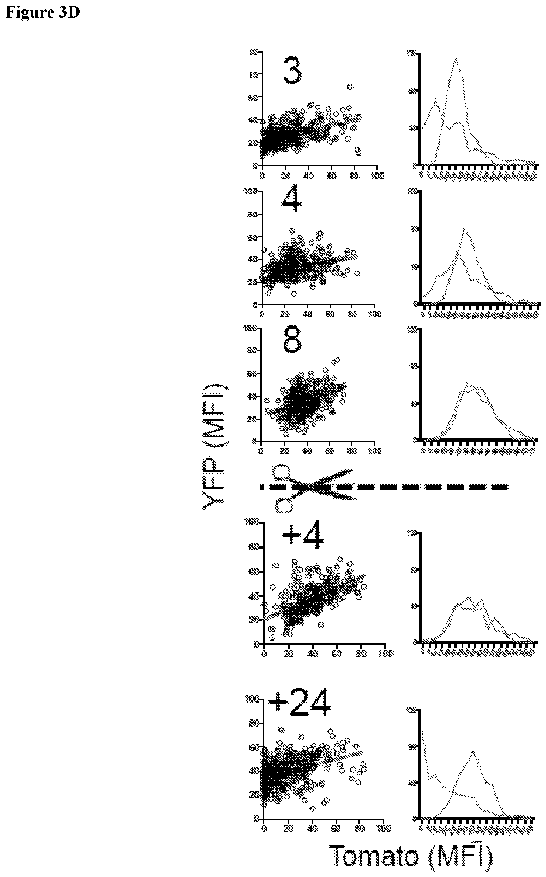

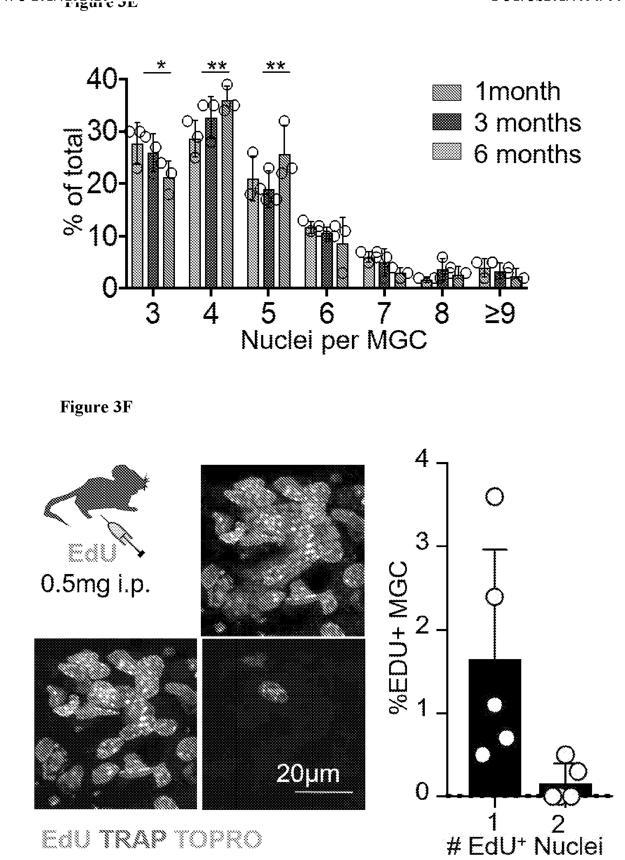

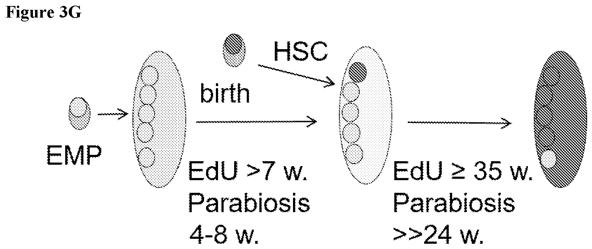

**Figures 3A-3G****.** *In vivo dynamics of osteoclasts.* **Figure 3A****:** *Parabiosis of Csf1r^{Cre};Rosa26^{LSL-YFP}* mouse surgically paired with a *Csf1r^{Cre};Rosa26^{LSL-tdTomato}* partner for 4 to 8 weeks. Representative (n=3) confocal microscopy image of frozen sections from the femur of a *Csf1r^{Cre};Rosa26^{LSL-YFP}* partner stained with antibodies for tdTomato (red) and YFP (green), ELF97 (blue) and TOPRO-3 (grey). **Figure 3B****:** Pie graphs represent the percentage of tdTomato positive (red), YFP positive (green) and double tdTomato/YFP positive cells (yellow) among BM mononuclear cells (MNC), Megakaryocytes (Mega) and Multinuclear Giant Cells (MGC), from parabionts paired for 1, 2, 3, 4, and 8 weeks(n=8). **Figure 3C****:** As in Figure 3B for parabionts separated after 4 weeks and analysed 14 weeks and 24 weeks after separation (n=3). **Figure 3D****:** Scatter plots represent the Mean Fluorescent Intensity (MFI) of individual TRAP+ MGC for YFP (Y axis) and tdTomato (X axis), and histograms represent the overlayed distribution of the MFI values for YFP and tdTomato in TRAP+ MGC at the indicated time-points. **Figure 3E****:** Bar graph display number of nuclei per TRAP+ MGC in femurs from wild type mice at 1, 3, and 6 month of age (n=3 mice per time point). **Figure 3F****:** Representative confocal microscopy image of an EdU-labeled nucleus in a TRAP+ osteoclast, and histogram displaying the percentages of TRAP+ osteoclast with EdU-labeled nuclei and the number of labeled nuclei per cell 72 hrs after intravenous pulse-labeling with EdU (n=5 mice). **Figure 3G****:** A model for development and maintenance of osteoclast syncytia. Data are mean ± SD, circles on graphs represent individual mice, n indicates the number of mice per group. Statistics: two-way ANOVA with Tukey's multiple comparisons test *p≤0.05 **p≤0.005.

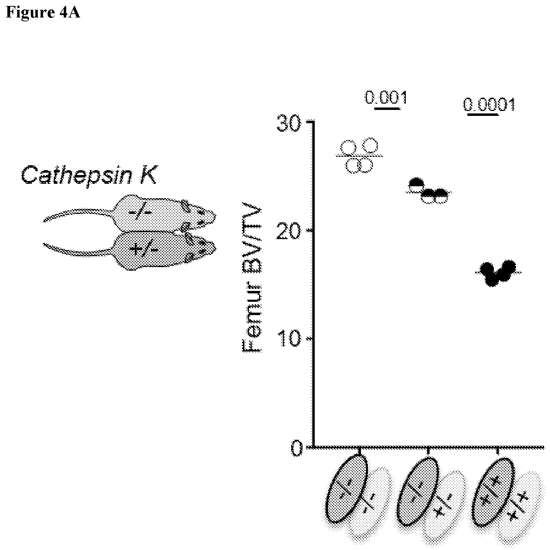

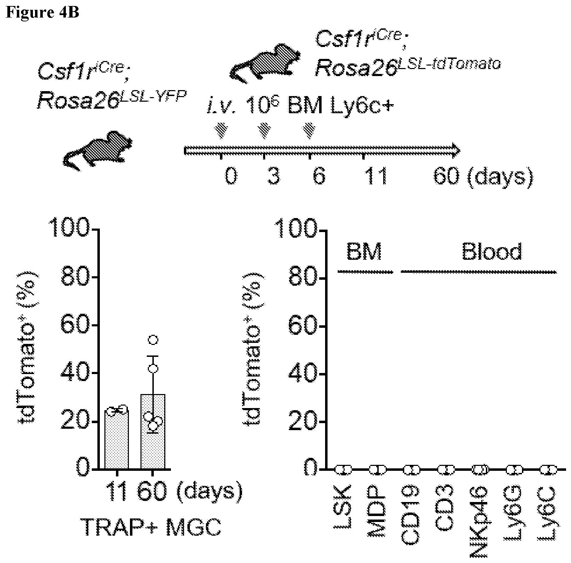

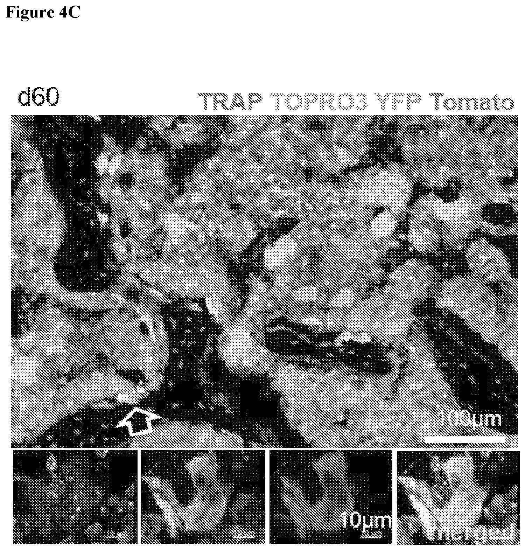

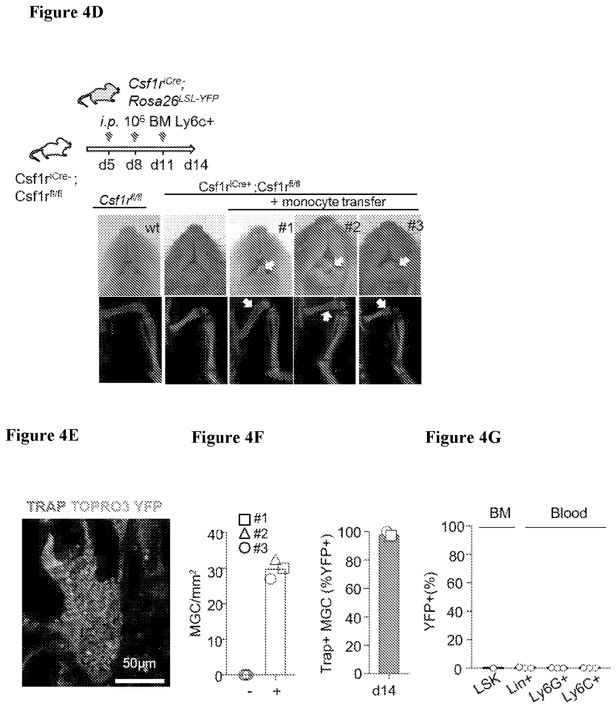

**Figures 4A-G.** *Rescue of Osteopetrosis.* **Figure 4A****:** Bone volume over total volume (BV/TV%) of femurs from 10 week-old Cathepsin K^{-/-} mice (n=3) after 6 weeks parabiosis with Cathepsin K^{+/-} heterozygote mice and from positive (n=4) and negative (n=4) controls parabionts, analysed by Von Kossa staining. **Figure 4B****:** Monocyte transfer: histograms represent percentages of tdTomato+ cells among bone TRAP+ MGC from *Csf1r^{Cre};Rosa26^{LSL-YFP}* recipients analysed by confocal microscopy (*left*) 11 days (n=2) and 60 days (n=5) after i.v. transfer at age 6 week-old of 3×10⁶ bone marrow Ly6C⁺ cells from *Csf1r^{Cre};Rosa26^{LSL-tdTomato}* donors, and percentages of tdTomato+ cells among bone marrow precursors and blood leukocytes analyzed by flow cytometry after 60 days (n=5, *right*). **Figure 4C****:** Representative high power confocal microscopy image of the femur of a recipient mouse 60 days after i.v. transfer (from Figure 4B), stained with antibodies for tdTomato and YFP, and ELF97 and TOPRO-3. Figure **4D****:** Representative photographs of teeth, and CT scan of leg bones from *Csf1r^{Cre}; Csf1r*^{*fl*/*fl*} mice (n=3) transferred with monocytic cells from *Csf1r^{Cre}; Rosa26^{LSL-YFP}* donors at post-natal day 5, 8, and 11, and from wt and non-transferred *Csf1r^{Cre}; Csf1r*^{*fl*/*fl*} controls. Arrows indicated the presences of teeth eruption (upper panel) and bone marrow cavity (lower panel). **Figure 4E****:** Representative confocal microscopy image of femur from mice #3 in Figure 4D, stained with antibodies for YFP, ELF97 and TOPRO-3. **Figure 4F****:** Histograms represent (*Left*) the number of TRAP+ osteoclasts number in bone sections from mice in Figure 4D and non-transferred controls and (*Right*) the percentage of TRAP+ cells that are YFP+ in transferred mice. Symbols represents individual mice, values are average for 3 sections per mice. At least n = 100 osteoclasts were quantified per mice. **Figure 4G****:** Histograms represent percentages of YFP+ cells among bone marrow precursors and blood leukocytes in the recipient mice (in Figure 4D) at the time of analysis. Data are mean ± SD, circles on graphs represent individual mice, n indicates the number of mice per group. Statistical significance was analyzed with GraphPad Prism using ANOVA with Tukey's multiple comparisons test ***p < 0.001; ****p < 0.0001.

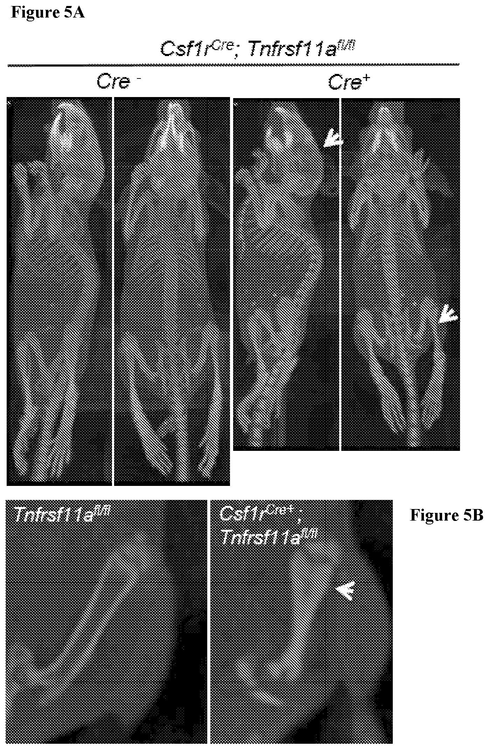

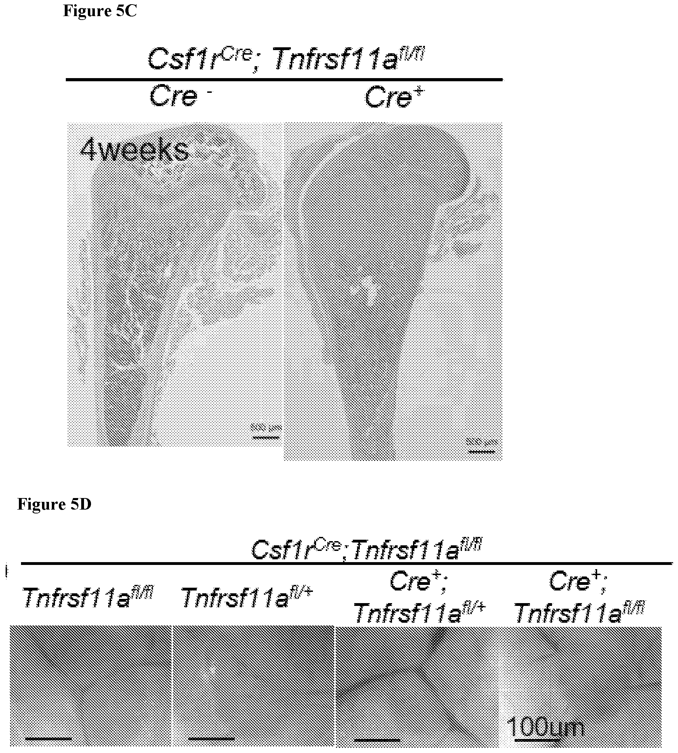

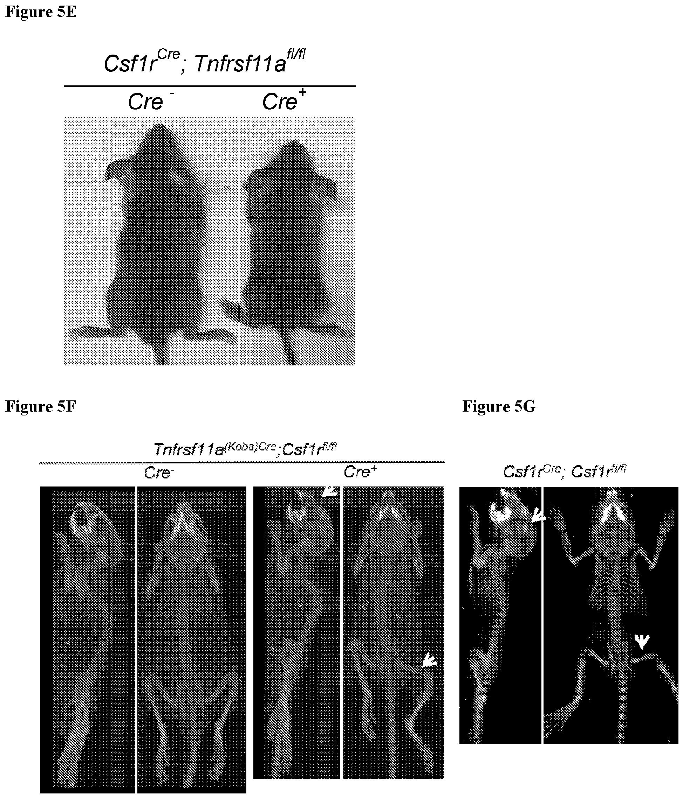

**Figures 5A-G.** *Csf1r^{Cre}; Tnfrsf11a*^{*fl*/}*^{fl}, Csf1r*^{*fl*/}*^{fl};Csf1r^{Cre}, and Tnfrsf11a^{Cre}; Csf1r*^{*fl*/}*^{fl} mice are osteopetrotic.* **Figures 5A****-B:** Representative Computed Tomography scans (nanospect CT) of 4 week old *Csf1r^{Cre};Tnfrsf11a*^{*fl*/*fl*} mice arrows point skull deformation, and absence of bone marrow cavity in mutant mice. **Figure 5C****:** H&TRAP staining of bone sections of 4 week old *Csf1r^{Cre};Tnfrsf11a*^{*fl*/*fl*} mice showing closure of the bone marrow. Histology of paraffin sections (5µm thickness) corroborates the phenotype from mice in Figure 5B. **Figure 5D****:** Representative photograph of inguinal lymph nodes in *Csf1r^{Cre}; Tnfrsf11a*^{*fl*/*fl*} mice. **Figure 5E****:** Representative photograph of *Csf1r^{Cre}; Tnfrsf11a*^{*fl*/*fl*} and littermates controls. **Figure 5F****:** Representative computed tomography scan reconstructions (nanospect CT) of 4 weeks-old Tnfrsf11a^{(Koba)Cre}; *Csf1r*^{*fl*/*fl*} mice. Arrows point to skull deformation, but the presence of bone marrow cavity in mutant mice, as in Figures 5A, B. **Figure 5G****:** Representative Computed Tomography scans of *Csf1r^{Cre}; Csf1r*^{*fl*/*fl*} mice. Arrows shows skull deformation, and absence of bone marrow cavity in mutant mice as in A,B,. CT scans and photographs are representative of >10 litters.

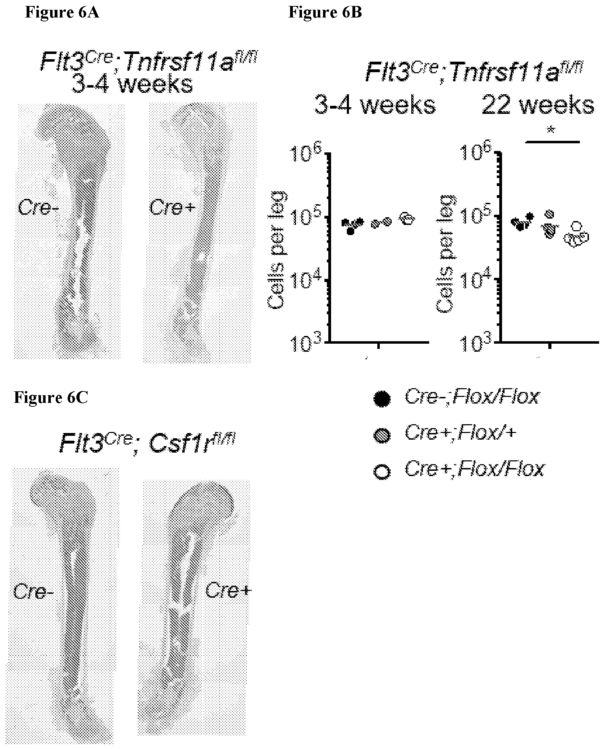

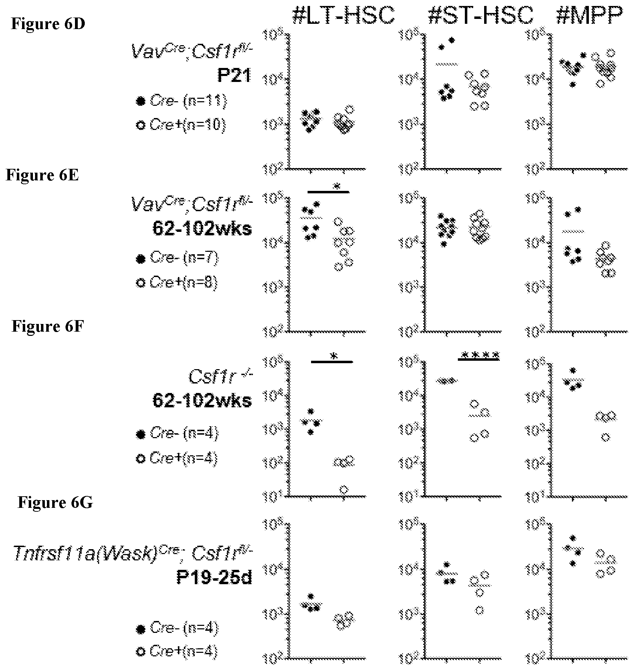

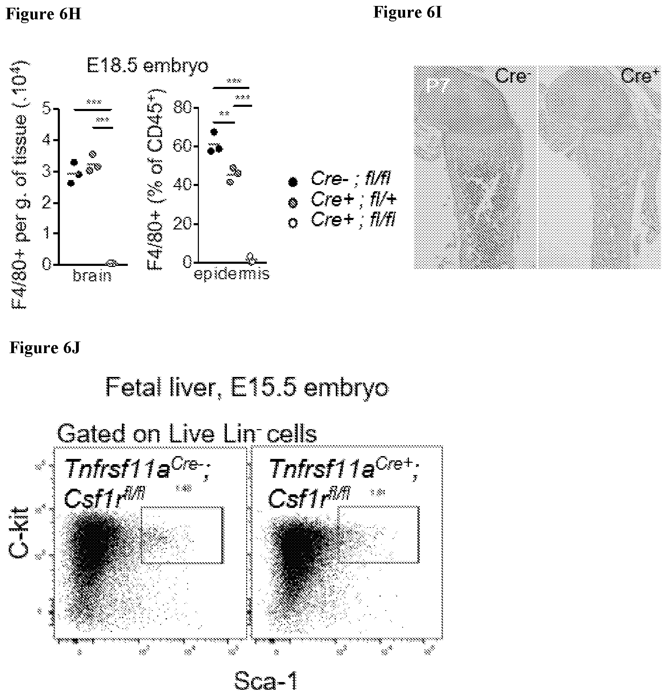

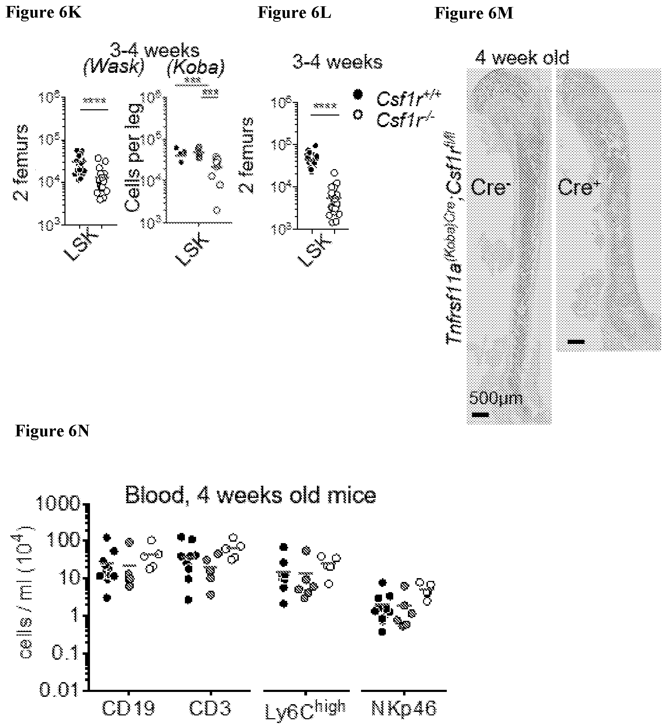

**Figures 6A-N.** *Bone histology and Flow cytometry analysis of bone marrow phenotypic KSL, LT-HSCs, ST-HSCs, and MPPs in mice of indicated genotypes.* **Figure 6A****:** Young Flt3^{Cre};Csf1r^{fl/fl} and Flt3^{Cre};Tnfrsf11a^{fl/fl}mice have normal long bone: H&TRAP staining of bone sections from 4 week old *Flt3^{Cre};Tnfrsf11a*^{*fl*/*fl*} mice showing normal bone structure and bone marrow cavity. **Figure 6B****:** LSK cell numbers in bone marrow *Flt3^{Cre}; Tnfrsf11a*^{*fl*/*fl*} (n=3), *Flt3^{Cre}; Tnfrsf11a*^{*fl*/+} mice (n=2), and littermate controls (n=4) in 3-4 week old mice and *Flt3^{Cre}; Tnfrsf11a*^{*fl*/*fl*} (n=5), *Flt3^{Cre}; Tnfrsf11a*^{*fl*/+} mice (n=4), and littermate controls (n=4) in 22 week old mice. **Figure 6C****:** H&TRAP staining of bone sections from 4 week old *Flt3^{Cre};Csf1r*^{*fl*/*fl*}mice showing normal bone structure and bone marrow cavity. **Figures 6D****, E:** Phenotypic long-term hematopoietic stem cells (LT-HSCs) are reduced in aged but not old Vav^{Cre};Csf1r^{fl/-} mice, (**Figures 6F****, G**) LT-HSC are reduced in in young Csf1r^{-/-} and to a lesser extent in young Tnfrsf11a*^{(Wask)Cre;}Csf1r*^{*fl*/*-*} Numbers per 2 femur are shown. **Figure 6H****:** Flow cytometry analysis of F4/80+ cells in brain (microglia) and epidermis (Langerhans cells) in E18.5 *Tnfrsf11a^{Cre}; Csf1r*^{*fl*/*fl*} embryos and littermate controls (n=3 per group). **Figure 61:** H&TRAP staining of bone sections from P7 *Tnfrsf11a^{Cre};Csf1r*^{*fl*/*fl*} and littermate control showing absence of the bone marrow cavity. **Figure 6J****:** Flow cytometry plot of Fetal liver at E15.5 (B/C representative results of 3 experiments). **Figure 6K****:** LSK numbers in bone marrow of 3-4 week old *Tnfrsf11a^{Cre(wask)};Csf1r*^{*fl*/*fl*} (n=24) and littermate controls (n=20) and *Tnfrsf11a^{Cre(koba)};Csf1r*^{*fl*/}*^{fl} (n=7), Tnfrsf11a^{Cre(koba)};Csf1r*^{*fl*+*l*} (n=6) and littermate controls (n=8). **Figure 6L****:** For comparison, LSK numbers in bone marrow of 3-4 week old *Csf1r^{-l-}* (n=22)and littermate controls (n=21). **Figure 6M****:** Representative micrographs of femur sections from 4 week old *Tnfrsf11a^{Cre};Csf1r*^{*fl*/*fl*} and littermate control stained with hematoxylin/TRAP. **Figure 6N****:** Blood leukocytes numbers in 4 week old *Tnfrsf11a^{Cre};Csf1r*^{*fl*/*fl*} mice (n=5), *Tnfrsf11a^{Cre};Csf1r*^{*fl*/+} mice (n=6) and littermate controls (n=12). Circles represent individual mice, results from 3 independent experiments. Data are mean ± SD, circles on graphs represent individual mice, n indicates the number of mice per group. Statistical significance was analysed with GraphPad Prism using unpaired two tailed t-tests. Significance was considered at p value (p) *p < 0.05; **p < 0.005; ***p < 0.0005; ****p < 0.0001. LT-HSC: Lin- Kit+ Sca-1+; Kit+ Sca1+ CD34- Flt3-. Phenotypic short-term hematopoietic stem cells (ST-HSC): Lin- Kit+ Sca-1+ CD34+ Flt3-. Multipotent progenitors (MPP): Lin- Kit+ Sca-1+ CD34+ Flt3+. Lin: CD3 CD19 NK1.1 Ter119 CD11b Gr1 B220.

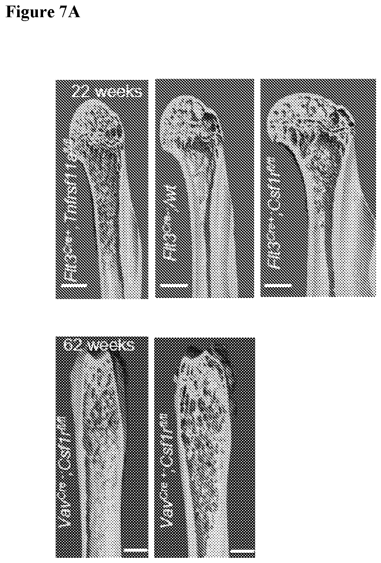

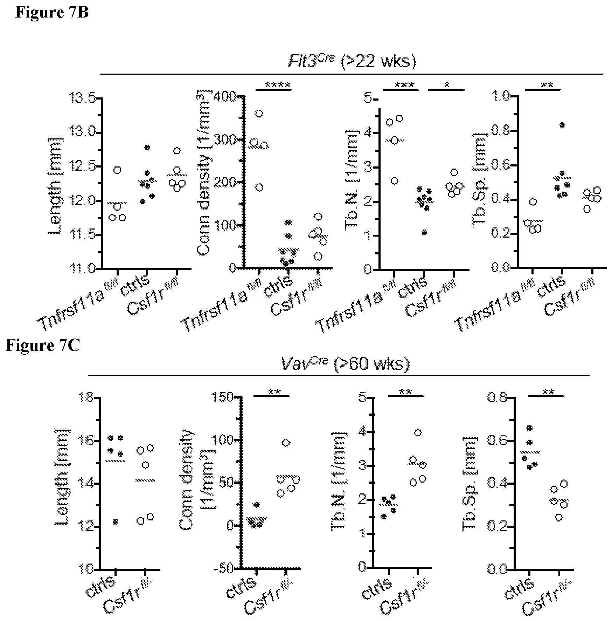

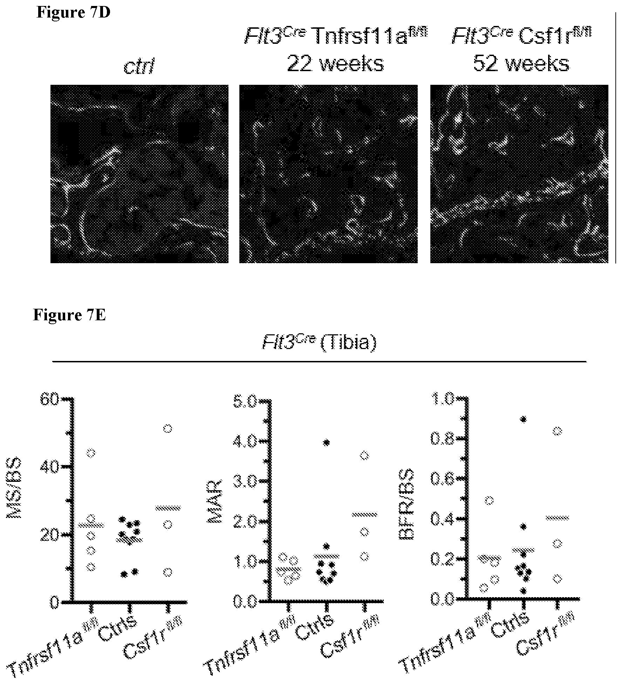

**Figures 7A-E.** *Bone histomorphometry in old Flt3^{Cre};Tnfrsf11a*^{*fl*/}*^{fl}, Flt3^{Cre};Csf1r*^{*fl*/*fl*}, *and Vav^{Cre};Csf1r*^{*fl*/}*^{fl}mice and control littermates.* **Figure 7A****:** Representative Micro CT pictures , genotype is indicated. **Figure 7B****:** Bone length, connectivity density (Conn density), Trabecular Number (Tb.N.), and Trabecular Spacing (Tb.Sp.) analyzed by microCT in aged *Flt3^{Cre}; Tnfrsf11a*^{*fl*/*fl*} (n=4), *Flt3^{Cre};Csf1r*^{*fl*/*fl*} (n=4) and control littermates (n=7). **Figure 7C****:** Bone histomorphometry as in Figure 7B, in *Vav^{Cre};Csf1r*^{*fl*/*fl*} mice and control littermates (n=5). **Figures 7D****, E:** Dynamic bone histomorphometry in aged Flt3^{Cre} ;Tnfrsf11a^{fl/fl}and Flt3^{Cre} ;Csf1r^{fl/fl} mice using *in vivo* calcein labeling. Representative micrographs of calcein labeling (green) of femura of mice from the indicated genotypes and ages (Figure 7D). Scale bars represent 200µm (top) and 50µm (bottom). Quantification of calcein labeling by fluorescence microscopy of mineralized surface/bone surface (MS/BS), mineral apposition rate (MAR), and bone formation rate/bone surface (BFR/BS) in aged *Flt3^{Cre}; Tnfrsf11a*^{*fl*/*fl*} (n=5), *Flt3^{Cre};Csf1r*^{*fl*/*fl*} (n=3) and control littermates (n=10) Data are mean ± SD, circles on graphs represent individual mice, n indicates the number of mice per group. Statistical significance was analysed with GraphPad Prism using unpaired two tailed t-tests. Significance was considered at p value (p) *p < 0.05; **p < 0.005; ***p < 0.0005; ****p < 0.0001.

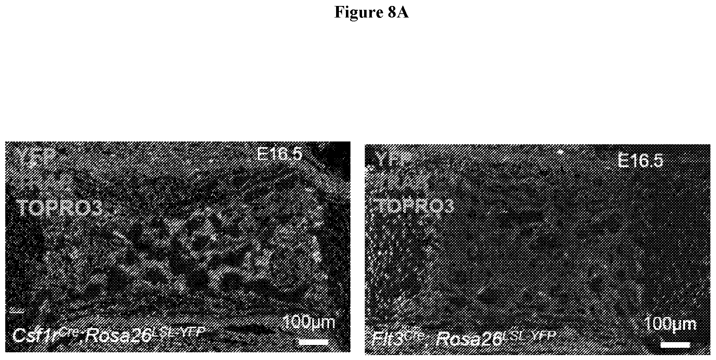

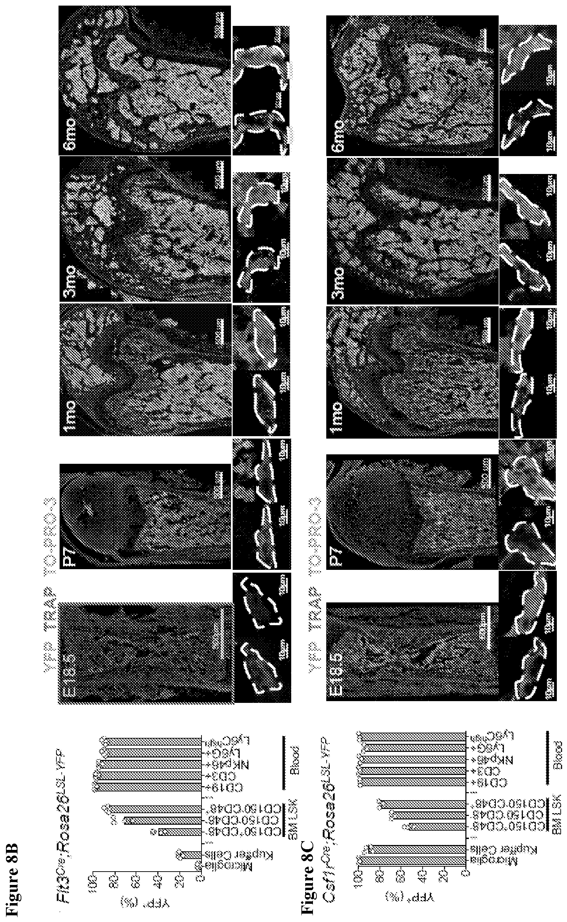

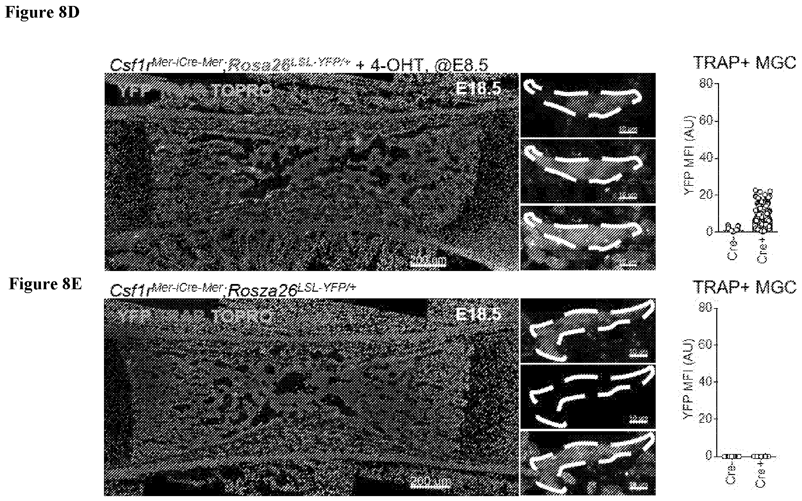

**Figures 8A-E.** *Colonization of the bone marrow by Csf1r*+ *and Flt3*+ *hematopoietic cells.* **Figure 8A****:** Representative confocal microscopy images of frozen sections from *Flt3^{Cre};Rosa26^{LSL-YFP}* and *Csf1r^{Cre};Rosa26^{LSL-YFP}*mice analyzed at E16.5 (n=3). **Figure 8B****:** YFP labeling efficiency in *Flt3^{Cre};Rosa26^{LSL-YFP}* mice analyzed by flow cytometry in the indicated cell populations, and by confocal microscopy on frozen bone sections at the indicated time (Right). Insets show YFP expression on individual osteoclasts. YFP: anti-GFP antibody, TRAP: ELF97 fluorescent substrate, TO-PRO-3: nuclear stain. **Figure 8C****:** YFP labeling efficiency in *Csf1r^{Cre};Rosa26^{LSL-YFP}*mice analyzed as in Figure 8A. Data from Figures 8A, B are representative of at least 3 experiments by time point and genotypes. Circles represent individual mice. Genetic lineage tracing of osteoclasts in ossification centers using *Csf1r^{MeriCreMer}; Rosa26^{LSL-YFP}.*Representative high power confocal microscopy images from embryonic femurs showing MGCs in primary ossification centers from *Csf1r^{MeriCreMer};Rosa26^{LSL-YFP}.* **Figure 8D****:** E18.5 embryos pulsed with 4-hydroxytamoxifen at E8.5 showing YFP expression in MGCs after Cre recombination and quantitated as MFI (right bar graph) from Cre+ (n=8) and Cre-(n=4), and **Figure 8E** unpulsed controls showing the lack of YFP in Cre+ (n=4) and Cre- (n=4). Sections were labeled with antibodies against YFP, TRAP (ELF97 substrate) and TO-PRO-3 as a nuclear stain.

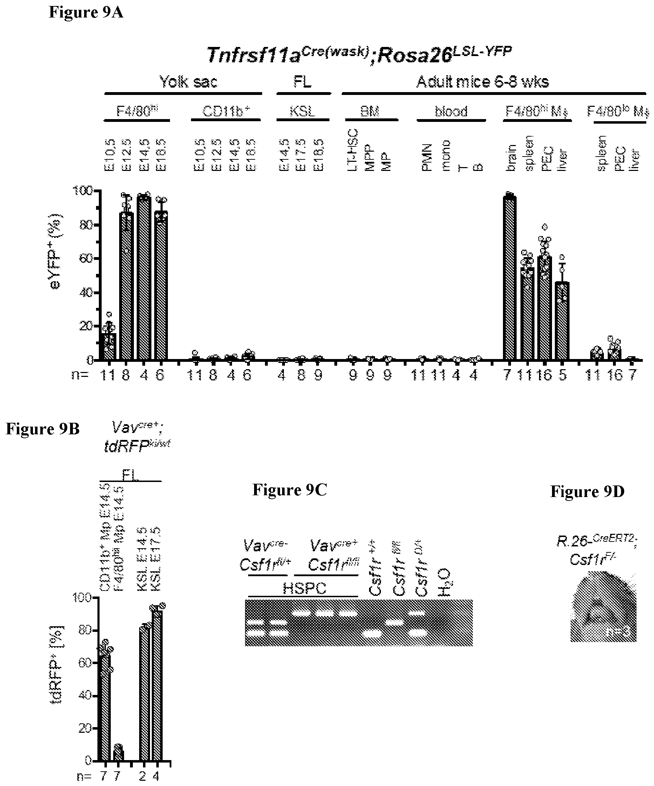

**Figures 9A-D.** *Tnfrs11a^{(wask)Cre} knock-in mice allow deletion of target genes in fetal macrophages, but not definitive HSC and their progeny in blood and tissues, while Vav^{cre} mice allow deletion of target genes in definitive HSC, but not fetal macrophages.* **Figure 9A****:** Bar graphs indicate percentages of eYFP expression by flow cytometry in cells from *Tnfrsf11a^{Cre};Rosa26^{LSL-YFP}* in the indicated cell types, organs, and time points. Data represent 3 independent experiments n numbers indicated on x axis. **Figure 9B****:** Lineage tracing in the fetal liver of *Vav*^{*cre*+}*;tdRFP*^{*wt*/*ki*} mice n numbers indicated on x axis. **Figure 9C****:** Representative molecular analysis of *Csf1r* deletion in purified bone marrow HSPC from 62 weeks old *Vav^{cre};Csf1r*^{*fl*/*fl*} mice and controls (n=5). **Figure 9D****:** Representative photograph of teeth from 3 week old *R.26-CreER*^{*T2*+}*;Csf1r*^{*fl*/*-*} pulsed with Tamoxifen at E10.5 (n=3 mice from 3 independent litters). FL: Fetal Liver, HSPC: hematopoietic stem and progenitor cell, LT-HSC: Long-term Hematopoietic Stem Cells, MPP: Multipotent progenitors, PMN: polymorphonuclear cells, mono: monocytes, T: T cells, B: B cells, PEC: peritoneal exudate cells. Data are mean ± SD, circles on graphs represent individual mice, n indicates the number of mice per group.

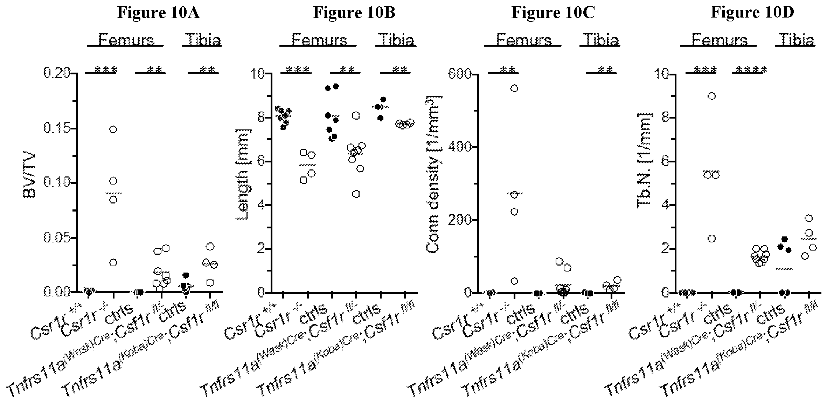

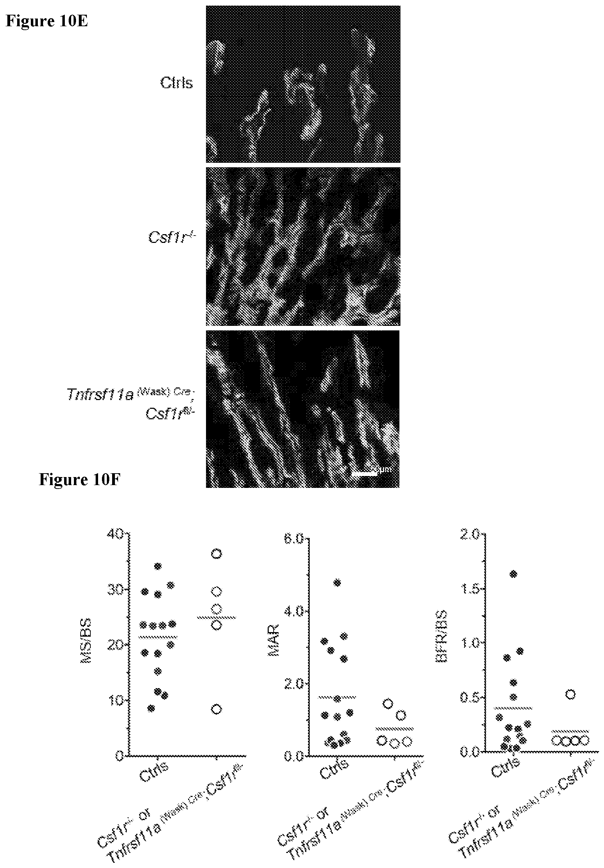

**Figures 10A-F.** *Bone morphometric and dynamic histomorphometry effects of the deletion of Csf1r in P21 Tnfrsf11a^{Cre}; Csf1r*^{*fl*/}*^{fl} mice.* Bone volume / total vlume (BV/TV, **Figure 10A****),** Bone length **(****Figure 10B****),** connectivity density (Conn density, **Figure 10C****),** and Trabecular Number (Tb.N., **Figure 10D****)** was analyzed by microCT in 21 day/old mice. *Csf1r*^{*-*/*-*}(n=4) and control littermates (n=7), *Tnftsf11a*^{*(wask)Cre*+}*;Csf1r^{fll-}*(n=8) and *Tnfrsf11a*^{*(wask)Cre*+}*;Csf1r*^{*fll*+}(n=7), *Tnfrsf11a*^{*(wask)Cre*+}*;Csf1r^{fll-}*(n=3) and control littermates (n=5). **Figure 10E****:** Representative micrographs of calcein labeling (green) of femur of mice from the indicated genotypes and ages (n=4). Scale bars represent 50µm. **Figure 10F****:** Quantification of calcein labeling by fluorescence microscopy: mineralized surface/bone surface (MS/BS), mineral apposition rate (MAR), and bone formation rate/bone surface (BFR/BS) in *Tnfrsf11a*^{*(wask)Cre*+}*;Csf1r^{fll-} Csf1r*^{*-*/*-*} (n=4) and control littermates (n=15). Data are mean ± SD, circles on graphs represent individual mice, n indicates the number of mice per group. For statistical analysis unpaired two-tailed t-test. *p≤0.05, **p≤0.005, ***p<_0.0005, ****p<0.0001.

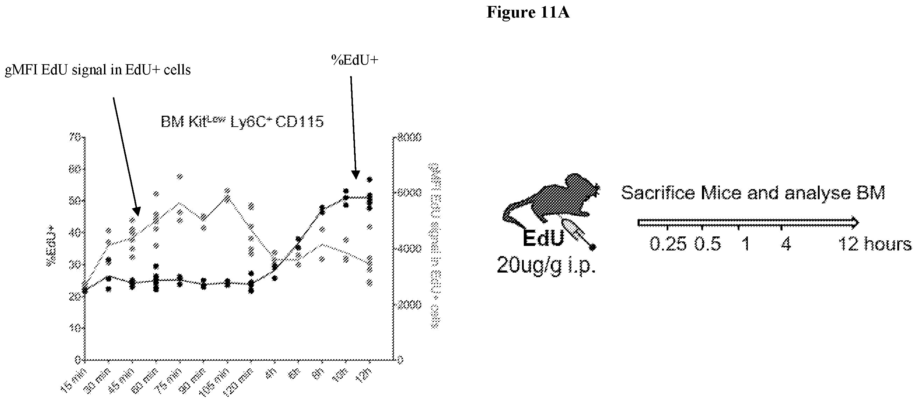

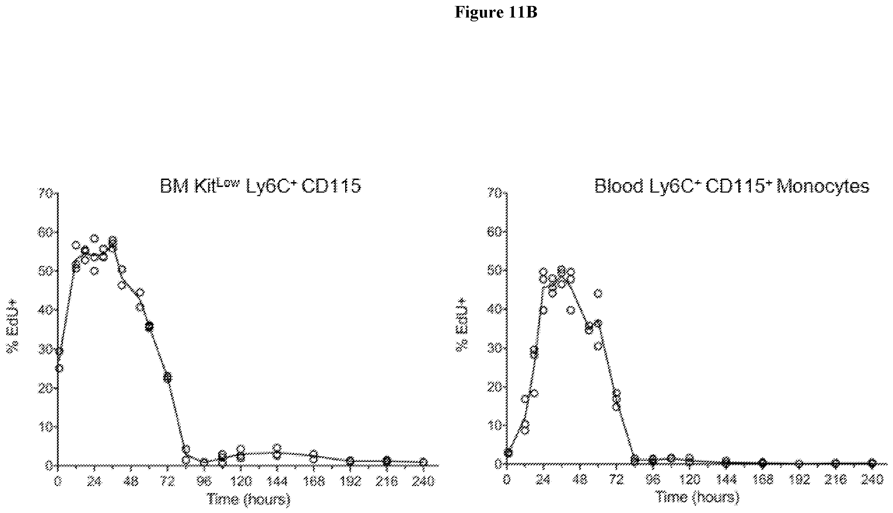

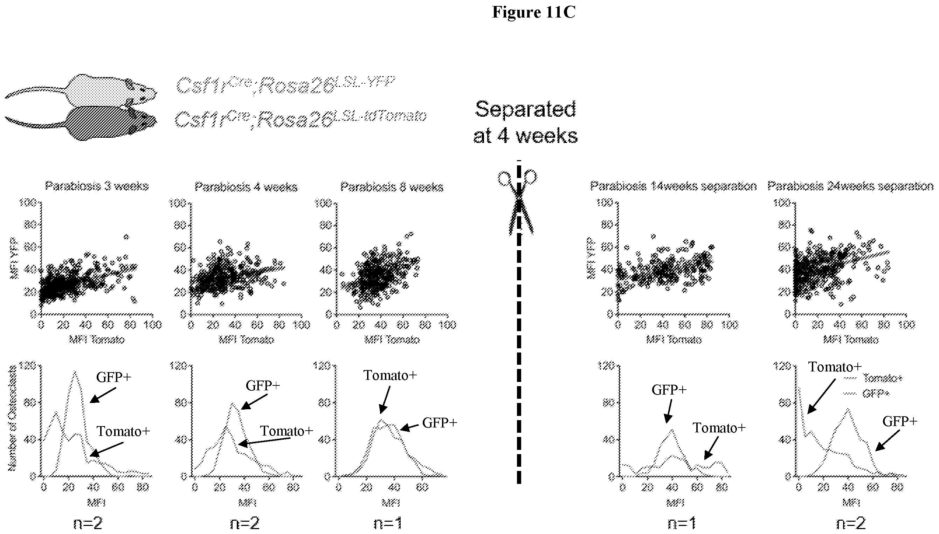

**Figures 11A-C.** *EdU labeling of bone marrow myeloid cells.* **Figure 11A****:** Short term kinetics: EdU (20µg/g) is injected intraperitoneally to C57Bl6/N mice at t=0 min. Mice are sacrificed at the indicated time points and the % of EdU+ cells (blue) and the geometric MFI of EdU+ cells (red) and determined by flow cytometry, showing rapid EdU incorporation. % of EdU+ cells plateau at ~30 min, and gMFI plateau at ~ 75 minutes. Following a first round of cell division, -50% of monocytic cells are labeled after 8 to 12 hrs (n=3-8 mice see source data). **Figure 11B****:** Long term kinetics: (1-240hrs) EdU is injected as in Figure 11A and % of EdU+ monocytic cells in bone marrow (top) and blood (bottom) is determined by flow cytometry, showing that labeling of ~50% of monocytic cells is observed for -2 days. Labeled cells are not detectable after 3 days. Circles represent determination from individual mice, data for each time point are pooled from 2-3 independent experiments see source data. **Figure 11C****:** Parabiosis between *Csf1r^{Cre};Rosa26^{LSL-YFP} and Csf1r^{Cre};Rosa26^{LSL-tdTomato}* pairs as described in (Figure 3), paired for 1-8 weeks, and from *Csf1r^{Cre};Rosa26^{LSL-YFP}* partners separated after 4 weeks of parabiosis and analyzed 4 weeks, 14 weeks and 24 weeks after separation. Scatter plots represent the Mean Fluorescent Intensity (MFI) of individual TRAP⁺ MGC for YFP (Y axis) and tdTomato (X axis), and histograms represent the overlaid distribution of the MFI values for YFP and tdTomato in TRAP⁺ MGC at the indicated time-points. Data are mean ± SD, circles on graphs represent individual mice, n indicates the number of mice per group.

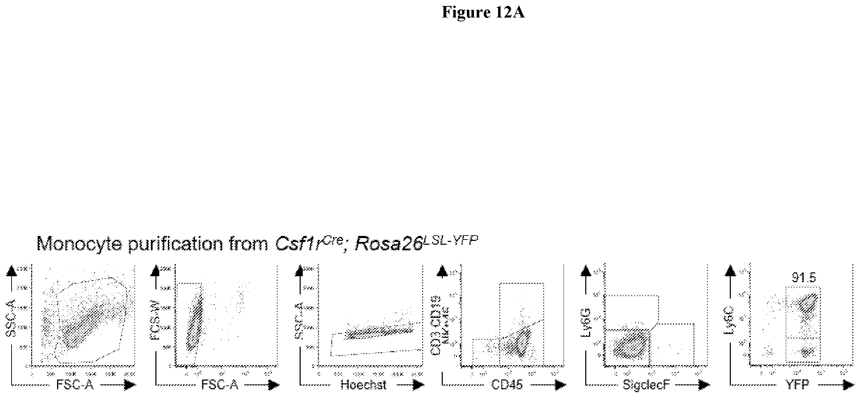

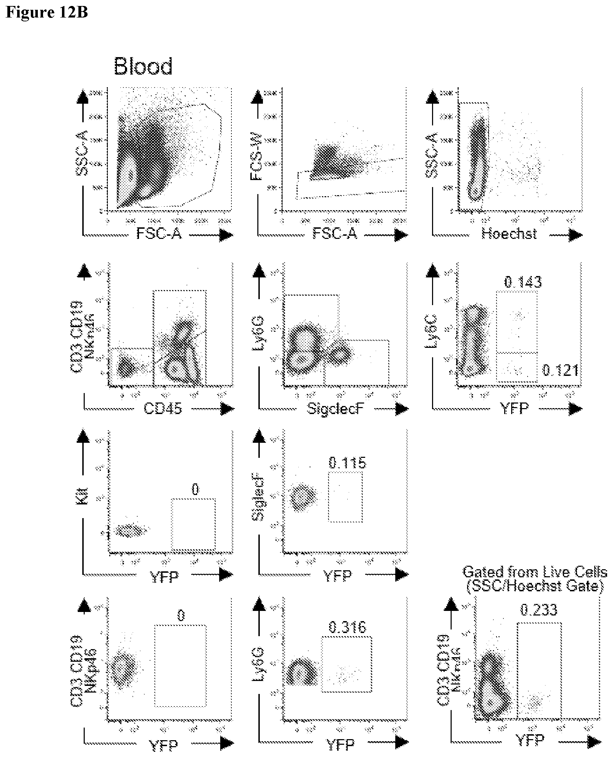

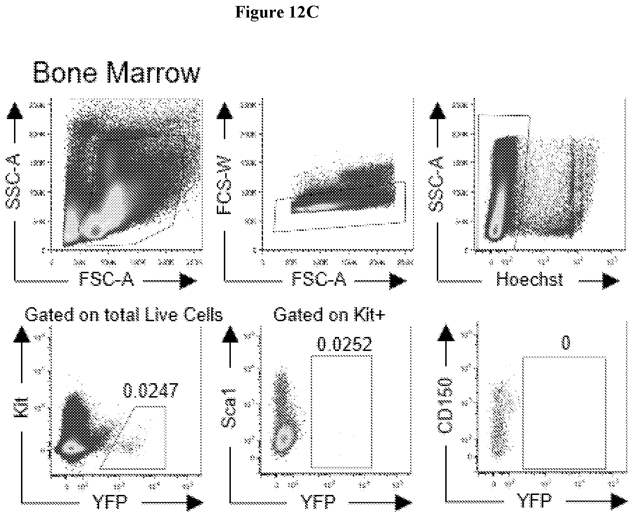

**Figures 12A-C.** *FACS analysis of monocyte purification, and blood* / *bone marrow from transferred Csf1r^{Cre}; Csf1r*^{*fl*/}*^{fl} mice.* **Figure 12A****:** Representative flow cytometry plots of purified bone marrow monocytes from magnetic bead based enrichment (MACS), percentage of live YFP+ monocytes is indicated. Representative flow cytometry plots from blood **(****Figure 12B****)** and bone marrow **(****Figure 12C****)** of 14 day old mice transferred with 1E6 YFP+ monocytes on day 5,8,11, the percentage of YFP+ cells is indicated. Experiments A-C are representative of 3 independent experiments.

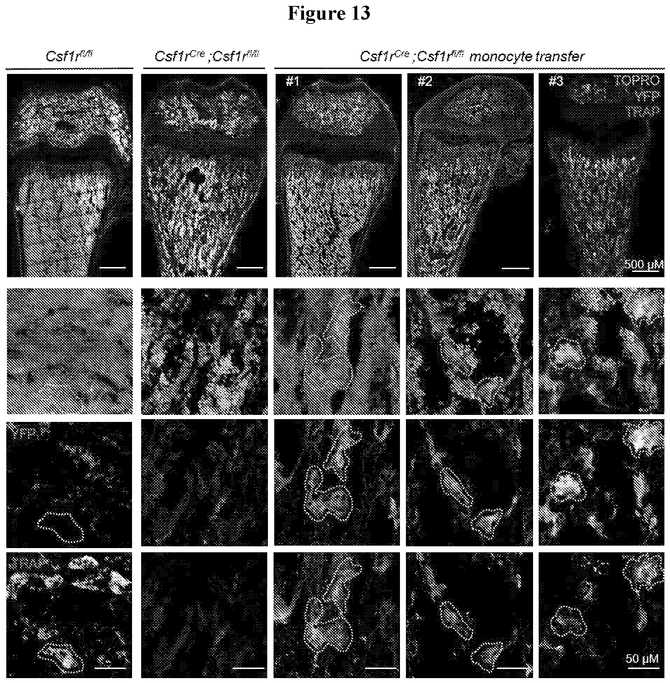

**Figure 13****.** *Rescue of osteoclasts by monocyte transfer in Csf1r^{Cre}; Csf1r*^{*fl*/}*^{fl} mice.* High power confocal microscopy images of frozen sections from *Csf1r^{Cre}; Csf1r*^{*fl*/*fl*} mice transferred with monocytes from *Csf1r^{Cre} Rosa26^{LSL-YFP}* and controls, stained with antibodies for YFP, TRAP substrate ELF97 and TOPRO-3 as a nuclear stain. Examples of multinucleated Trap+, YFP+ cells (osteoclasts) are indicated with dotted lines. n=3 mice from independent litters. Numbers (#1,2,3) correspond to the mice in Figure 4D.

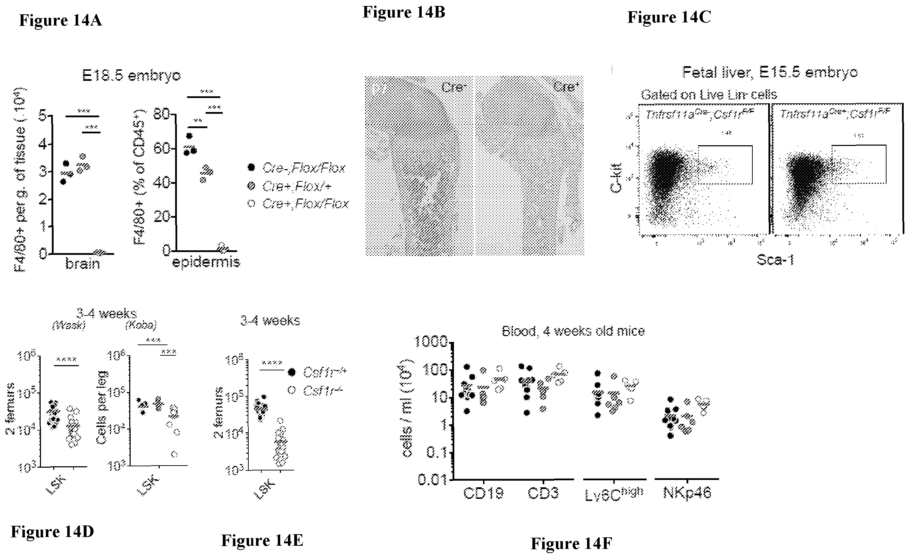

**Figures 14A-F.** *Csf1r deletion in Tnfrsf11a^{Cre} Csf1r*^{*F*/}*^{F} mice results in lack of tissue macrophages and osteoclasts at birth, while definitive HSC and their progeny are present in the fetal liver and blood.* **Figure 14A****:** Flow cytometry analysis of F4/80+ cells in brain (microglia) and epidermis (Langerhans cells) in E18.5 *Tnfrsf11a*^{*Cre*+}*;Csf1r*^{*F*/*F*} embryos and littermate controls. **Figure 14B****:** H&TRAP staining of bone sections from P7 *Tnfrsf11a*^{*Cre*+}*;Csf1r*^{*F*/*F*} and *Tnfrsf11a^{Cre-;}Csf1r*^{*F*/*F*} littermate control showing absence of the bone marrow cavity. **Figure 14C****:** Flow cytometry plot of Fetal liver at E15.5. **Figure 14D** LSK numbers in bone marrow of 3-4 week old *Tnftsf11a*^{*Cre*+}*^{;}Csf1r*^{*F*/*F*} and littermate controls. **Figure 14E****:** For comparison, LSK numbers in bone marrow of 3-4 week old *Csf1r*^{*-*/*-*} and littermate controls. **Figure 14F****:** Blood leukocytes numbers in 4 week old *Tnftsf11a*^{*Cre*+}*^{;}Csf1r*^{*F*/*F*} mice and littermate controls. Circles represent individual mice, results from 3 independent experiments.

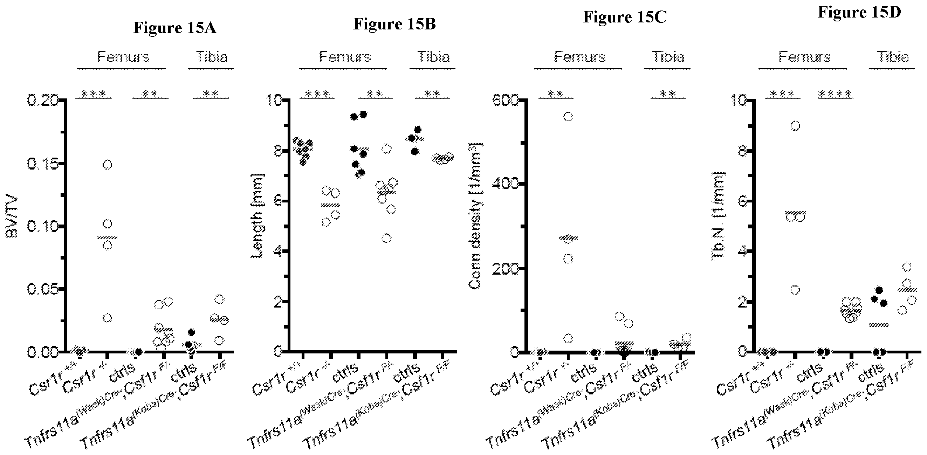

**Figures 15A-D.** *Bone morphometric effects of the deletion of Csf1r in P21 Tnfrsf11ac*^{*Cre*+}*;Csf1r*^{*F*/}*⁻ or* ^{*F*/}*^{F} mice.* Bone volume / total vlume (BV/TV, **Figure 15A****),** Bone length **Figure 15B****,** connectivity density (Conn density, **Figure 15C****),** and Trabecular Number (Tb.N., **Figure 15D****)** was analyzed by microCT in 21 day/old mice of indicated genotype. Controls (ctrls) included *Tnfrsf11a(wask)*^{*Cre*+}*;Csf1r*^{*F*/+} or *Tnfrsf11a*^{*(wask)Cre*+}*;Csf1r^{FlF}* and *Tnfrsf11a*^{*(koba)Cre*+}*;Csf1r*^{*F*/+} genotypes. Circles represent 1 mouse with 3-7 mice per group. For statistical analysis unpaired two-tailed t-test. *p<_0.05, **p≤0.005, ***p≤0.0005, * * * *p<_0.0001.

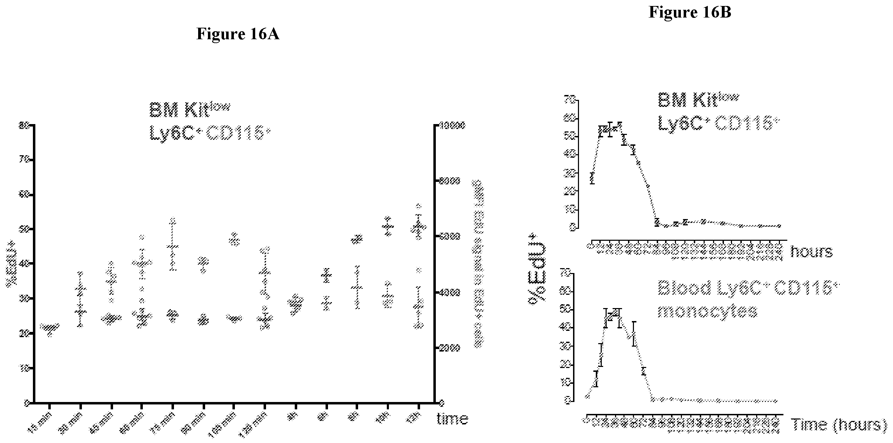

**Figures 16A-B.** *EdU labeling of bone marrow myeloid cells.* **Figure 16A****:** Short term kinetics: EdU (20µg/g) is injected intraperitoneally to C57B16/N mice at t=0 min. Mice are sacrificed at the indicated time points and the % of EdU+ cells (blue) and the geometric MFI of EdU+ cells (red) and determined by flow cytometry, showing rapid EdU incorporation. % of EdU+ cells plateau at ~30 min, and gMFI plateau at ~ 75 minutes. Following a first round of cell division, -50% of monocytic cells are labeled after 8 to 12 hrs. **Figure 16B****:** Long term kinetics: (1-240hrs) EdU is injected as in (A) and % of EdU+ monocytic cells in bone marrow (top) and blood (bottom) is determined by flow cytometry, showing that labeling of -50% of monocytic cells is observed for -2 days. Labeled cells are not detectable after 3 days. Circles represent determination from individual mice, data for each time point are pooled from 2-3 independent experiments.

### DETAILED DESCRIPTION

It is to be appreciated that certain aspects, modes, embodiments, variations and features of the present technology are described below in various levels of detail in order to provide a substantial understanding of the present technology. The definitions of certain terms as used in this specification are provided below. Unless defined otherwise, all technical and scientific terms used herein generally have the same meaning as commonly understood by one of ordinary skill in the art to which this present technology belongs.

### I. Definitions

The following terms are used herein, the definitions of which are provided for guidance.

As used herein, the singular forms "a", "an" and "the" include plural referents unless the content clearly dictates otherwise. For example, reference to "a cell" includes a combination of two or more cells, and the like.

As used herein, the term "about" and the use of ranges in general, whether or not qualified by the term about, means that the number comprehended is not limited to the exact number set forth herein, and is intended to refer to ranges substantially within the quoted range while not departing from the scope of the invention. As used herein, "about" will be understood by persons of ordinary skill in the art and will vary to some extent on the context in which it is used. If there are uses of the term which are not clear to persons of ordinary skill in the art given the context in which it is used, "about" will mean up to plus or minus 10% of the particular term.

A "chimeric nucleic acid" comprises a coding sequence or fragment thereof linked to a nucleotide sequence that is different from the nucleotide sequence with which it is associated in cells in which the coding sequence occurs naturally.

As used herein, the terms "effective amount," or "therapeutically effective amount," and "pharmaceutically effective amount" refer to a quantity sufficient to achieve a desired therapeutic and/or prophylactic effect, e.g., an amount which results in the prevention of a disease, condition, and/or symptom(s) thereof. In the context of therapeutic or prophylactic applications, the amount of a composition administered to the subject will depend on the type and severity of the disease and on the characteristics of the individual, such as general health, age, sex, body weight, and tolerance to the composition drugs. It will also depend on the degree, severity, and type of disease or condition. The skilled artisan will be able to determine appropriate dosages depending on these and other factors. In some embodiments, multiple doses are administered. Additionally or alternatively, in some embodiments, multiple therapeutic compositions or compounds are administered. In the methods described herein, compositions comprising the monocytic cells of the present technology, may be administered to a subject having one or more signs, symptoms, or risk factors of osteopetrosis, including, but not limited to stunted growth, skeletal deformity, increased likelihood of bone fracture, anemia, recurrent infections, hepatosplenomegaly, facial paralysis, abnormal cortical bone morphology, abnormal form of vertebral bodies, abnormal temperature regulation, abnormality of the ribs, abnormality of vertebral epiphysis morphology, bone pain, cranial nerve paralysis, craniosynostosis, hearing impairment, and hypocalcemia. For example, a "therapeutically effective amount" of the compositions of the present technology, includes levels at which the presence, frequency, or severity of one or more signs, symptoms, or risk factors of osteopetrosis are, at a minimum, ameliorated. In some embodiments, a therapeutically effective amount reduces or ameliorates the physiological effects of osteopetrosis, and/or the risk factors of osteopetrosis, and/or the likelihood of developing osteotpetrosis. In some embodiments, a therapeutically effective amount is achieved by multiple administrations. In some embodiments, a therapeutically effective amount is achieved with a single administration.

The term "engineered" is used herein to refer to a cell or organism that has been manipulated to be genetically altered, modified, or changed, e.g., by disruption of the genome. For example, an "engineered monocytic cell" refers to a monocytic cell that has been manipulated to be genetically altered, modified, or changed. For example, in some embodiments, an engineered monocytic cell refers to a monocytic cell that has been transduced with a lentivirus designed to express a nucleotide sequence of interest, e.g., a cDNA coding for the wild type allele of any one or more of *CA2, CLCN7, CTSK, CSF1R, IKBKG, ITGB3, OSTM1, PLEKHM1, TCIRG1, TNFRSF11A,* or *TNFSF11* under a strong promoter.

"Heterologous nucleic acid" refers to a nucleic acid, DNA, or RNA, which has been introduced into a cell (or the cell's ancestor), and which is not a copy of a sequence naturally found in the cell into which it is introduced. Such heterologous nucleic acid may comprise segments that are a copy of a sequence that is naturally found in the cell into which it has been introduced, or fragments thereof.

As used herein, "prevention," "prevent," or "preventing" of a disorder or condition refers to, in a statistical sample, reduction in the occurrence or recurrence of the disorder or condition in treated subjects/samples relative to an untreated controls, or refers delays the onset of one or more symptoms of the disorder or condition relative to the untreated controls.

As used herein "subject" and "patient" are used interchangeably and refer to a mammalian subject. In some embodiments, "subject" means any animal (mammalian, human, or other) patient that can be afflicted with osteopetrosis and when thus afflicted is in need of treatment. In some embodiments, the subject is a human.

"Treating," "treat," "treated," or "treatment" of a disease or disorder includes: (i) inhibiting the disease or disorder, i.e., arresting its development; (ii) relieving the disease or disorder, i.e., causing its regression; (iii) slowing progression of the disorder; and/or (iv) inhibiting, relieving, or slowing progression of one or more symptoms of the disease or disorder.

It is to be appreciated that the various modes of treatment or prevention of medical diseases and conditions as described are intended to mean "substantial," which includes total but also less than total treatment or prevention, and wherein some biologically or medically relevant result is achieved.

### II. General

Described herein are compositions and methods for the treatment of osteopetrosis. Taken together, the data described herein identify: (i) the developmental origin of osteoclasts, the cells that shape bone architecture; (ii) a mechanism that controls the maintenance of osteoclast function after birth; and (iii) a novel strategy to treat osteopetrosis and to modulate osteoclast activity *in vivo.* In particular, the data described herein demonstrate that parabiosis or transfusion of monocytic cells results in long-term gene transfer in osteoclasts in the absence of HSC chimerism and can rescue an adult-onset osteopetrotic phenotype caused by cathepsin-K deficiency. Transfusion of monocytic cells is also sufficient to rescue bone development in early-onset autosomal recessive osteopetrosis in newborn mice.

### III. Osteopetrosis

Osteopetroses are a heterogeneous group of genetic disorders characterized by increased bone density due to impaired bone resorption by osteoclasts. The increased bone density places the affected individual at an increased risk for bone fracture. Normally, bone growth is a balance between osteoblasts (cells that create bone tissue) and osteoclasts (cells that destroy bone tissue). Individuals with osteopetrosis have a deficiency of osteoclasts, resulting in too little bone resorption and too much bone creation. The types of osteopetrosis are distinguished based on their pattern of inheritance: autosomal dominant, autosomal recessive, or X-linked.

The signs and symptoms of osteopetrosis may vary depending on the type of the disease, and mild forms of osteopetrosis may be asymptomatic. However, the typical signs and symptoms of osteopetrosis include: stunted growth, skeletal deformity, increased likelihood of bone fracture, anemia, recurrent infections, hepatosplenomegaly, facial paralysis, abnormal cortical bone morphology, abnormal form of vertebral bodies, abnormal temperature regulation, abnormality of the ribs, abnormality of vertebral epiphysis morphology, bone pain, cranial nerve paralysis, craniosynostosis, hearing impairment, and hypocalcemia.

The signs and symptoms of autosomal dominant osteopetrosis (ADO; also known as Albers-Schönberg disease), include multiple bone fractures, scoliosis, arthritis in the hips, and/or osteomyelitis. Autsomal recessive osteopetrosis (ARO) is often characterized by one or more of a high risk of bone fracture resulting from minor bumps or falls, pinched nerves in the head and face, impaired bone marrow function, slow growth, short stature, dental abnormalities, hepatosplenomegaly, intellectual disability, and epilepsy. Individuals diagnosed with intermediate autosomal osteopetrosis (IAO), which is a form of osteopetrosis that can have either an autosomal dominant or recessive pattern or inheritance, may be characterized by one or more of a high risk of bone fracture and anemia, calcifications in the brain, intellectual disability, and renal tubular acidosis. Individuals with the X-linked pattern of inheritance may be characterized by one or more of lymphedema, anhidrotic ectodermal dysplasia, and immunodeficiency.

Mutations in several genes have been linked to the various forms of osteopetrosis or may underlie the development of osteopetrosis. These include mutations in *CA2*, *CLCN7*, *CTSK, CSF1R, IKBKG, ITGB3, OSTM1, PLEKHM1, TCIRG1, TNFRSF11A* (which encodes for receptor activator of NF-xB (*RANK*)), and *TNFSF11* (which encodes for receptor activator of NF-κB ligand (*RANKL*)). Many of the genes associated with osteopetrosis are involved in the formation, development, and function of osteoclasts.

### IV. Therapeutic Applications

In some embodiments, a method for treating or preventing osteopetrosis in a subject in need thereof, comprising administering a composition comprising a therapeutically effective amount of monocytic cells from a healthy donor to the subject is provided. In some embodiments, the subject is characterized by decreased expression of one or more genes implicated in or potentially underlying the development of osteopetrosis, such as *CA2*, *CLCN7, CTSK, CSF1R*, *IKBKG, ITGB3, OSTM1, PLEKHM1, TCIRG1, TNFRSF11A*, and *TNFSF11.* In some embodiments, the subject is characterized by a cathepsin K deficiency.

In some embodiments, a method for treating or preventing osteopetrosis in a subject in need thereof, comprising administering to the subject a composition comprising a therapeutically effective amount of monocytic cells engineered to express one or more genes implicated in or potentially underlying the development of osteopetrosis, such as *CA2*, *CLCN7, CTSK, CSF1R, IKBKG, ITGB3, OSTM1, PLEKHM1, TCIRG1, TNFRSF11A*, and *TNFSF11* is provided. In some embodiments monocytic cells used for treatment are genetically modified to correct a genetic abnormality or to improve or changed cellular functioning according to known genetic engineering protocols. In some embodiments, a method of treating or preventing osteopetrosis in a subject comprises: (a) obtaining a sample of monocytic cells from the subject; (b) genetically correcting one or more mutations in the monocytic cells, (c) culturing the monocytic cells; and (d) providing the corrected monocytic cells to the subject. In some embodiments, a method for treating or preventing osteopetrosis comprises: (a) obtaining a sample of monocytic cells from the subject; (b) genetically engineering the cells to express a nucleotide sequence coding for the wild type allele of any one or more *of CA2, CLCN7, CTSK, CSFIR, IKBKG, ITGB3, OSTM1, PLEKHM1, TCIRG1, TNFRSF11A,* and *TNFSF11* with a suitable transducing vector, such as a lentiviral vector; (c) culturing the engineered monocytic cells under conditions sufficient to express the nucleotide sequence; (d) removing the viral particles from the engineered monocytic cells; and (e) providing the engineered monocytic cells to the subject. In some embodiments, the transducing vector encoding the one or more of *CA2, CLCN7, CTSK, CSFIR, IKBKG, ITGB3, OSTM1, PLEKHM1, TCIRG1, TNFRSF11A,* and *TNFSF11* is a mammalian expression vector. In some embodiments, the mammalian expression vector is a lentiviral vector or transposon vector.

Subjects suffering from osteopetrosis can be identified by any or a combination of diagnostic or prognostic assays known in the art. For example, typical symptoms of osteopetrosis include, but are not limited to, stunted growth, skeletal deformity, increased likelihood of bone fracture, anemia, recurrent infections, hepatosplenomegaly, facial paralysis, abnormal cortical bone morphology, abnormal form of vertebral bodies, abnormal temperature regulation, abnormality of the ribs, abnormality of vertebral epiphysis morphology, bone pain, cranial nerve paralysis, craniosynostosis, hearing impairment, and hypocalcemia.

For therapeutic applications, a composition comprising a therapeutically effective amount of monocytic cells from a health donor and/or monocytic cells engineered to express one or more genes selected from *CA2, CLCN7, CTSK, CSF1R, IKBKG, ITGB3, OSTM1, PLEKHM1, TCIRG1, TNFRSF11A,* and *TNFSF11* is administered to the subject. In some embodiments, the composition is administered according to any acceptable transfusion regimen. In some embodiments, the composition is administered one, two, three, four, or five times per day. In some embodiments, the composition is administered more than five times per day. Additionally or alternatively, in some embodiments, the composition is administered every day, every other day, every third day, every fourth day, every fifth day, or every sixth day. In some embodiments, the composition is administered weekly, bi-weekly, tri-weekly, or monthly. In some embodiments, the composition is administered for a period of one, two, three, four, or five weeks. In some embodiments, the composition is administered for six weeks or more. In some embodiments, the composition is administered for twelve weeks or more. In some embodiments, the composition is administered for a period of less than one year. In some embodiments, the composition is administered for a period of more than one year or until a desired therapeutic outcome is observed in the subject.

In some embodiments, treatment of subjects diagnosed with or suspected of having osteopetrosis with one or more compositions of the present technology ameliorates or eliminates one or more of the following symptoms of osteopetrosis: stunted growth, skeletal deformity, increased likelihood of bone fracture, anemia, recurrent infections, hepatosplenomegaly, facial paralysis, abnormal cortical bone morphology, abnormal form of vertebral bodies, abnormal temperature regulation, abnormality of the ribs, abnormality of vertebral epiphysis morphology, bone pain, cranial nerve paralysis, craniosynostosis, hearing impairment, and hypocalcemia.

### Prophylactic Methods

In one aspect, the present technology provides a method for preventing or delaying the onset of osteopetrosis or one or more symptoms of osteopetrosis in a subject at risk of having or developing osteopetrosis. In prophylactic applications, compositions of the present technology are administered to a subject susceptible to, or otherwise at risk of for osteopetrosis in an amount sufficient to eliminate or reduce the risk, or delay the onset of the disease, including biochemical, histologic and/or behavioral symptoms of the disease, its complications and intermediate pathological phenotypes presenting during development of the disease.

Administration of a prophylactic compositions can occur prior to the manifestation of symptoms characteristic of the disease or disorder, such that the disease or disorder is prevented or, alternatively, delayed in its progression.

### EXAMPLES

The following examples are provided by way of illustration only and not by way of limitation. Those of skill in the art will readily recognize a variety of non-critical parameters that could be changed or modified to yield essentially the same or similar results. The examples should in no way be construed as limiting the scope of the present technology, as defined by the appended claims.

### Materials and Methods

*Csf1r^{iCre}, Csf1r^{MeriCreMer}, Csf1r^{flox}* were kindly provided by Dr Jeffrey Pollard, *Csf1r*^{*-* /-} from Richard Stanley, Flt3^{Cre} were kindly provided by Dr Thomas Boehm, Myb^{+/-} mice were kindly provided by Dr John Frampton, and *Vav^{Cre}* were kindly provided from Dr Thomas Graf. *Tnfrsf11a^{flox}* mice were kindly provided by JM Penninger, *Tnfrsf11a*^{*Koba*/*Cre*} mice were kindly provided by Dr Yasuhiro Kobayashi, *Ctsk^{tm1(cre)Ska}* mice were kindly provided by Dr. Ostrowsky (MSKCC) and *Tnfrsf11a*^{*Wask*/*Cre*} mice were genereated in the Waskow Lab. *Rosa.26-CreER^{T2}* (*R26-CreER^{T2}*) were kindly provided from Drs. Pierre Chambon and Anton Berns. *Rosa26^{LSL-YFP}* (stock number: 006148) and *Rosa26^{LSL-tdTomato}* (stock number: 007908) reporter mice were purchased from The Jackson Laboratory.

*Animal procedures.* Mice were bred and kept under specific pathogen conditions in separated ventilated cages in the animal facility of MSKCC and the Medical Theoretical Center of the TU Dresden. All experiments with osteopetrotic mice that lack teeth were performed with mice maximal 4 weeks of age that were kept with the lactating mother or provided with DietGel 76A (Clear H²O, 72-07-5022) to avoid secondary effects from malnutrition. Experiments were performed in adherence to the Institutional Review Board (IACUC 15-04-006) from MSKCC and Landesdirektion Dresden and were in compliance with relevant ethical regulations. Mice greater than 7 days old were sacrificed by cervical dislocation (TU Dresden), CO₂ asphyxiation or anesthesia (MSKCC). To harvest embryos, pregnant females were sacrificed and embryos were collected by postmortem cesarean from the uterus and exsanguinated through decapitation in cold PBS (Fisher, 14190).

*Genotyping:* PCR genotyping was performed according to protocols described previously and indicated in **Table 1.** The investigators were not blinded to allocation during experiments and outcome assessment.

**Table 1.**

| **Mouse line** | | **Primer 1 sequence 5' --> 3'** | **Primer 2 sequence 5' --> 3'** | **Denaturation** | **Annealing** | **Elongation** | **Cycles** | **Final Elongation** | **Expected bands** |
|---|---|---|---|---|---|---|---|---|---|
| *Csf1r^{iCre}* | Mutant allele | TCTCT GCCCA | CTCTG ACAGA | 94°C - 45sec | 60°C - 45sec | 72°C - 45sec | 30 | 72°C - 5 min | Mutant: 400 bp |
| | | GAGTC ATCCT | TGCCA GGACA | | | | | | |
| *CSf1r^{MeriC reMer}* | WT allele | TCGAA GCTTG CATGC CTGCA | TCTCT GCCCA GAGTC ATCCT | 94°C - 45sec | 60°C - 90sec | 72°C - 90sec | 30 | 72°C - 5 min | WT: 1Kb |
| | Mutant allele | TCATT CCAGA ACCAG AGC | GATCG TGTTG GGGAA GCC | | | | | | Mutant: 2 Kb |
| *Csf1r^{flox}* | Mutant allele | GCCAC CATGT GTCCG TGCTT | ACCCA GAGCC CCCAC AGATA | 94°C - 30sec | 60°C - 60sec | 72°C - 60sec | 35 | 72°C - 2 min | WT: 450 bp Floxed: 500 bp |
| *Csf1r*^{-/-} | Mutant allele | GGTGG ATGTG GAATG TGTGC G | CGTTT CTTGT GGTCA GGGTG C | 95°C - 20sec | 62°C - 20sec | 72°C - 40sec | 35 | 72°C - 5 min | Mutant: 314 |
| *Flt3^{Cre}* | Mutant allele | ACGGA GTCCA GGCAA CTTCC | GAAGC ATGTT TAGCT GGCCC | 94°C - 30sec | 58°C - 35sec | 68°C - 60sec | 44 | 72°C - 2 min | Mutant: 400 bp |
| *Myb*^{*+*/*-*} | WT allele | CCATG CGTCG CAAGG TGGAA C | GTGCT TCGGC GATGT GGTAA | 94°C - 30sec | 60°C - 30sec | 72°C - 60sec | 35 | 72°C - 7 min | WT: 200 bp |
| | Mutant allele | CCATG CGTCG CAAGG TGGAA C | TGGCC GCTTTT CTGGA TTCAT C | | | | | | Mutant: 300 bp |
| *Rosa.26^{Cr} eERT2* | Mutant allele | GCCTG CATTA CCGGT CGATG CAACG A | AATGG TGTTG CCGCG CCATC TGCCA C | 95°C - 20sec | 67°C - 20sec | 72°C - 50sec | 40 | 72°C - 2 min | Mutant: 700bp |
| *Rosa26^{LS} L-YFP* | WT allele | AAGTC GCTCT GAGTT GTTAT | GGAGC GGGAG AAATG GATAT G | 94°C - 40sec | 60°C - 60sec | 72°C - 60sec | 34 | 72°C - 5 min | WT: 525 bp |
| | Mutant allele | AAGTC GCTCT GAGTT GTTAT | GCGAA GAGTT TGTCC TCAAC C | | | | | | Mutant: 300 bp |
| *Rosa26^{LS} L-tdTomato* | WT alelle | AAGGG AGCTG CAGTG GAGTA | CCGAA AATCT GTGGG AAGTC | Step 1: 94°C - 20sec | 65°C - 15sec (0.5C decreas e per cycle) | 68°C - 10sec | 10 | 72°C - 2 min | WT: 297 bp Mutant: 196 bp |
| | Mutant allele | GGCAT TAAAG CAGCG TATCC | CTGTT CCTGT ACGGC ATGG | Step 2: 94°C - 15sec | 60°C - 15sec | 72°C - 10sec | 28 | | |
| *Tnfrsf11a flox* | WT alelle | AGTGT GCCTG GCATG TGCAG ACCTT | CTGGT GGTTG TTCTCC TGGTG TCAT | 94°C - 30sec | 60°C - 30sec | 72°C - 40sec | 35 | 72°C - 5 min | WT: 256 bp Floxed: 390 bp Delta: 566 bp |
| | Mutant allele | AGTGT GCCTG GCATG TGCAG ACCTT | GGCAG AACTC GGATG CACAG ATTGG | | | | | | |
| *Tnfrsf11a KobalCre* | WT allele | TGAAG GGTGA CATCA TCGTG GT | AATAG GGGTG GGGTG ATA | 94°C - 30sec | 60°C - 30sec | 72°C - 30sec | 35 | 72°C - 5 min | WT: 530 bp Mutant: 274 bp |
| | Mutant allele | ACTTC TCCAT GGTAG CCTCC | AATAG GGGTG GGGTG ATA | | | | | | |
| *Tnfrsf11a* ^{*Wask*/*Cre*} | Mutant allele | AACCT GAGGA TGTGA GGGAC TA | GTCAA AGTCA GTGCG TTCAA AG | 95°C - 30sec | 58°C - 30sec | 68°C - 60sec | 35 | 72°C - 2 min | Mutant: 210 bp |
| *Vav*^{*DD*/*Cre*} | Mutant allele | GCCTG CATTA CCGGT CGATG CAACG A | AATGG TGTTG CCGCG CCATC TGCCA C | 95°C - 20sec | 67°C - 20sec | 72°C - 50sec | 40 | 72°C - 2 min | Mutant: 700bp |

*Fate mapping with tamoxifen inducible Cre models.* For timing of embryonic development, mice were crossed at night, the following day a positive vaginal plug was considered as 0.5 days post-coitum (dpc), as previously described. Embryos were harvested as indicated below. For postnatal time point, pregnant females were monitored for the date of delivery, caesarean sections were carried out at term and neonates were fostered using lactating CD-1 females.

*Csf1r^{MeriCreMer}* female mice were crossed with male *Rosa26*^{*LSL-YFP*/*LSL-YFP*} mice. Cre recombination in *Csf1r^{MeriCreMer};Rosa26^{LSL-YFP}* embryos at embryonic day E8.5 was induced with a single dose of 4-hydroxytamoxifen (4-OHT) injected intraperitoneally in pregnant mothers at a dose of 75µg/g of body weight supplemented with 37.5 µg/g of progesterone as previously described. Cre-mediated recombination in *Rosa.26-CreER^{T2}* tamoxifen (TAM) was introduced to pregnant mother by a single TAM gavage (5mg) at E10.5, supplemented with progesterone (37.5µg/g body weight resolved in Sunflower seed oil, Sigma-Aldrich) was injected *i.p.* directly after gavage. To analyze newborn or 3 weeks old mice, caesarean sections were carried out at term and neonates were fostered using lactating CD-1 females.

*Parabiosis.* For cellular complementation female *Csf1r^{iCre}* mice were crossed to male *Rosa26*^{*LSL-YFP*/*YFP*} or *Rosa26*^{*LSL-tdTomato*/*tdTomato*} mice. *Csf1r^{iCre};Rosa26^{LSL-YFP}* and *Csf1r^{iCre};Rosa26^{LSL-tdTomato}* females were used for parabiosis. For rescue of *CathepsinK* activity 4 week old female Ctsk^{*Cre*/*Cre*} and control littermate mice were used for parabiosis. Parabionts were kept on Sulfamethoxazole/Trimethoprim (Sulfatrim) diet for up to 8 weeks. Ex-parabionts were separated after 4 weeks for cellular complementation and 6 weeks for rescue of *CathepsinK* activity.

*Surgical procedure, pre-operative procedure:* weight-matched female partner mice for parabiosis were caged together few days before surgery. One day before the surgery the fur from lateral sides of mice was carefully removed with a trimmer followed by depilatory cream (for 3 minutes) at the site of surgery, excess of fur was removed with a moist gauze pad. Left side partner is shaved on the right side and vice versa. This procedure was performed under Isofluorane inhalation anesthesia. Mice were fed with food supplemented with Sulfatrim *ad libitum* one day prior to surgery.

*Surgery:* Mice were anesthetized intraperitoneally with 150mg/kg of ketamine and 15mg/kg of xylazine. Sterile eye lubricant (Paralube Vet Ointment, 17033-211-38) was applied to both eyes to prevent corneal drying during surgery. Following confirmation that a suitable anesthetic plane (no response to stimulation) has been attained, mice were placed in a supine position on a surgical tray with heat support provided by a heating pad. The surgical site was cleaned 3 times with cotton swabs soaked in povidone-iodine (Betadine) then with 70% ethanol. Before surgery a volume of 0.2ml of anesthetic agent bupivacaine (Marcaine 0.25-0.5% solution) was applied locally. Surgery was performed by a longitudinal skin incision on the lateral side of mice, approximately 0.5cm above the elbow to 0.5cm below the knee joint. Mice were laid side-by-side in close contact and the ligaments of the two knees and elbows were sutured together using monofilament non-absorbable suture. Then, the skin incisions was closed by apposing and clipping skin to skin of the pair with 9 mm wound clips.

*Post-operative procedure:* immediately after surgery mice were injected subcutaneously with 2 mg/kg of meloxicam and 0.5mg/kg of buprenorphine and for a maximum of 48 hours postoperatively. Mice are provided with Sulfatrim and Hydrogel (Clear H₂O, 70-01-5022) in medicups. Wound clips were removed 14 days after surgery under Isofluorane anesthesia.

*Parabiosis separation procedure:* Mice were anesthetized and prepared for surgery as indicated above. Mice were separated at the site of parabiosis junction. Using scissors, the skin joining both mice is cut longitudinally. The sutures around the elbows and knees are cut and removed. The resulting wound is closed with 9 mm wound clips. Mice were injected subcutaneously with 2 mg/kg of meloxicam and 0.5mg/kg of buprenorphine and for a maximum of 48 hours postoperatively. Wound clips were removed 14 days after surgery.

*Analysis:* Bones were prepared for histology on frozen sections as detailed below, and stained with antibodies to detect fluorescent proteins: anti-GFP biotin, anti-RFP (Abcam), fluorescent TRAP staining and TO-PRO-3 as a nuclear stain. Histological sections of 15µm thickness were scanned by confocal microscopy at 1.5µm Z stacks. Sections were quantified for the number of TRAP⁺ multinuclear cells (more than 3 nuclei per cell) and their YFP and Tomato expression using Imaris in 3D view and individual Z stacks. Pictures of the region of interest (area=2mm²) were then generated in Tiff format and analyzed by ImageJ software using ROI manager to calculate the mean fluorescence intensity (MFI) of YFP and Tomato for individual osteoclasts. For rescue of *CathepsinK* activity, dissected femurs were fixed in 10% neutral buffered formalin for 24 hours. Undecalcified bones were embedded in methyl methacrylate resin, and 7-µm sections were prepared on a rotation microtome. For mineralized bone volume over the total volume % (BV/TV%), sections were de-plastified and stained with von Kossa reagent (1% Silver nitrate/ Sodium formamide/ 5% Sodium thiosulfate) counterstained with Van Gieson solution.

*EdU pulse labeling.* 12 week old C57BL/6N mice (Charles River) were injected intraperitoneally with 25 µg/g of a 2.5mg/mL solution of EdU prepared extemporaneously (Fisher C10420, Click-iT EdU Alexa Fluor 488 Flow Cytometry Assay Kit). Blood, bone marrow, and bone samples were collected at 60hrs and 72hrs post injection and bone were prepared for histology of frozen sections as indicated above for adult mice.

*Histology of frozen sections.* Frozen sections were cut at 15µm thickness using cryofilm and stained overnight with rat anti-Tubulin (Abcam, ab6160, 1:200) and with secondary goat anti-rat Alexa Fluor 555 (ThermoFisher Scientific A21430), as described below. Sections were washed 3 times with PBS and stained for EdU using Click-iT EdU Alexa Fluor 488 Imaging Assay Kit (Thermo Fisher Scientific, C10337). Click-it reaction: 357.5µL of 1X Click-it reaction buffer, 40µL of CuSO₄ solution (100mM), 2.5µL of Alexa Fluor 488 azide solution and 100 µL of Reaction buffer additive for 500µL. Sections are incubated for 45 minutes at room temperature with 100µL of Click-iT EdU reaction buffer then washed with PBS and stain for fluorescent TRAP (ELF 97) and TO-PRO-3 (nuclear stain). Mounting media was 75% glycerol in PBS.

*Microscopy.* Images were acquired using an inverted Zeiss LSM880 laser scanning confocal microscope. Histological sections of 15µm thickness were tile-scanned at 1.0µm Z stacks.

*Analysis.* Sections were analyzed using Imaris in 3D view and individual Z stacks to quantify the percentage of EdU⁺ TRAP⁺ multinuclear cells and EdU⁺ nuclei per individual osteoclasts.

*Adoptive transfers.* Bone marrow monocytes were isolated from 12-16 weeks old donor. In fate-mapping experiments 1×10⁶ total cells from *Csf1r^{iCre};Rosa26^{LSL-tdTomato}* mice were transferred at day 0, day 3 and day 6 by retro-orbital injections into recipient *Csf1r^{iCre};Rosa26^{LSL-YFP}* age and gender matched mice. Mice were sacrificed at day 11 and day 60 after the first transfer. For rescue of osteoclast activity 1×10⁶ total bone marrow monocytes from *Csf1r^{iCre};Rosa26^{LSL-YFP}* or *Csf1riCre;Rosa26^{LSL-tdTomato}* were transferred intra-peritoneal at p5, p8, and p11. Mice were sacrificed at p14 5 days after the last transfer. Bone marrow and blood was analyzed for the percentage of chimerism. To enrich Ly6C⁺ cells the Monocyte Isolation Kit (BM) for mouse (MACS Miltenyi Biotec, 130-100-629) was used as indicated by the manufacturer. Cell numbers of Ly6C⁺ cells were calculated by determining the cell number / ml using a Neubauer chamber in combination with a staining for Ly6C analyzed by flow cytometry. Bone samples were dissected and prepared for frozen sections as described above and stained for anti-GFP, anti-RFP, fluorescent TRAP and TO-PRO-3. The percentages of YFP⁺ or Tomato⁺ multinuclear cells was quantified in femurs of recipients.

*Preparation and analysis of bone for histology of paraffin embedded samples.* Bone samples were fixed in 4% formaldehyde (Fisher, 28908) in PBS for 1 day (embryo) or 3 days (post-natal mice) at 4°C then washed 3 times with PBS and decalcified (for mice older than P7) in a 14% EDTA pH7.1 solution at 4°C for 5 days to 15 days, washed 3 times with PBS and dehydrated in 70% ethanol for 1 day and processed for paraffin sections. Longitudinal sections of femurs were cut at 5µm thickness using a Leica RM2265 paraffin microtome then place in *Superfrost* microscope slides let dry for 48 hours and heated in a dry incubator at 65°C for 1 hour, dewaxed and stained for TRAP and hematoxylin.

*Tartrate Resistant Acid Phosphatase (TRAP) staining protocol.* To stain for TRAP, slides were placed in coplin jars and incubated in a 1% (v/v) Naphtol-Ether substrate solution in basic stock solution for 1hr at 37°C followed by incubation in a solution containing 2% (v/v) sodium nitrate solution and 2% (v/v) basic fuchsin solution in basic stock solution for 20 mins at 37°C. Slides are rinse in 3 changes of water then stained with hematoxylin solution (Sigma, GHS332) diluted 1:4 in water for 2 minute, washed 3 times with water then dehydrated and mounted in Entellan (Millipore, 107960). *Solutions for TRAP staining:. Basic stock solution:* 0.92% (w/v) anhydrous sodium acetate (Sigma, S8750), 1.14% (w/v) dibasic dihydrate sodium tartrate (Sigma, S4797) and 0.28% (v/v) glacial acetic acid (Sigma, 537020) in distilled water, pH was adjusted between 4.7-5.0 with 5M sodium hydroxide (Fisher, S318-1). *Napthol Ether substrate solution*: 2% (w/v) Napthol AS-BI Phosphate (Sigma, 70482) in 2-Ethoxyethanol (Sigma, 256374). *Sodium nitrate solution:* 4% (w/v) Sodium nitrate (Sigma, 237213) in water. *Basic fuchsin solution:* 5% (w/v) basic fuchsin dye (Sigma, 857343) in 2N HCL (Fisher, A144-500).

Individual images from histological sections of 5µm thickness were acquired using a Zeiss Axio Lab.A1 light microscope with a N-Achroplan 2.5X/0.07 M27 (420920-9901) or a N-Achroplan 20X/0.45 M27 (420950-9901) objective. Pictures were taken in ZEN lite software and exported as tiff files. Panoramic images were created with the photo-merge tool in Adobe Photoshop CS6. Pictures of mice were acquired with a dissecting microscope Leica M80 equipped with a Leica IC80 HD camera at 1.0X magnification. The region of interest analyzed was the methaphyseal trabecular bone 2 mm below the growth plate. The numbers of TRAP⁺ multinuclear cells (more than 3 nuclei per cell), associated to bone tissue, were quantified in ImageJ using the Cell Counter plugin. Numerical values were plotted using GraphPad Prism. For Static and dynamic histomorphometry. Young and aged mice were injected (i.p.) twice with 15 mg/kg body weight Calcein (Sigma) dissolved in 1.4% NaHCO3/PBS 2 and 3 days apart, respectively. Mice were sacrificed 2 days after the last calcein injection. Femora and tibiae were fixed in 4% PBS-buffered paraformaldehyde and dehydrated in an ascending ethanol series. Subsequently, bones were embedded in methacrylate and cut into 7 µm sections to assess the fluorescent calcein labels. Unstained sections were analyzed using fluorescence microscopy to determine the mineralized surface/bone surface (MS/BS), the mineral apposition rate (MAR), and the bone formation rate/bone surface (BFR/BS).To determine numbers of osteoclasts, bones were decalcified for one week using Osteosoft (Merck), dehydrated, and embedded into paraffin. Tartrate-resistant acid phosphatase staining was used to assess the osteoclast surface per bone surface (Oc.SBS) and number of osteoclasts per bone surface (N.Oc/BS). Bone sections were analyzed using the Osteomeasure software (Osteometrics, USA) following international standards.

*Preparation of and analysis of bones for immunofluorescence on frozen sections.* Samples were prepared as above and after decalcification and washing were soaked in 30% sucrose in PBS at 4°C for 1-2 days. Tissue samples were placed in disposable histology plastic molds and embedded in FSC22 Frozen Section Compound Clear (Leica, 3801480) and placed on a flat surface of dry ice to let freeze.

*Immunofluorescence.* Bones were cut at 15µm thickness using a Cryostat Leica CM3050S with high profile microtome blades (Leica Surgipath DB80 HS) and cryofilm (Section Lab Inc.,) and let to dry for 48 hours at 4°C. Before staining with antibodies, sections were let to equilibrate at room temperature for 30 minutes, rehydrated with PBS (Fisher, 14190) 3 times for 5 min at room temperature. Sections were incubated with blocking buffer containing 0.25% BSA (Fisher BP1600), 10% normal goat serum (Life Technologies, PCN 5000) and 0.3% triton (Sigma, T8787) in PBS for 1hr at room temperature. Sections were washed 2 times with PBS for 5 minutes. Sections stained with anti-GFP biotin antibody were first incubated with Biotin/Streptavidin blocking kit (Vector laboratories, SP2002). Streptavidin blocking solution is prepared by adding 4 drops of streptavidin solution to 1mL of PBS/0.25% BSA, samples were incubated for 15 min, then washed once with PBS for 5 min. Biotin blocking solution is prepared by adding 4 drops of biotin solution to 1mL of PBS/0.25% BSA, samples were incubated for 15 min then washed once with PBS for 5 min. *Primary and secondary antibodies* used are listed in **Table 2.** Sections were also stained with fluorescent TRAP and nuclear stain.

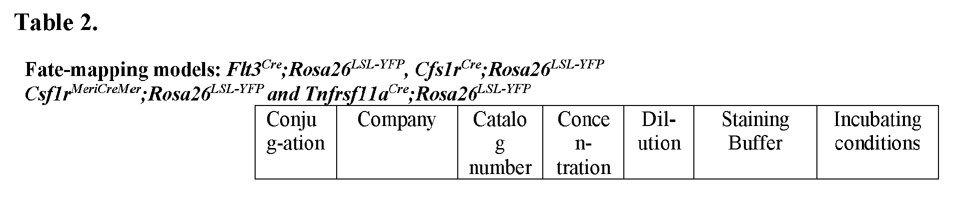

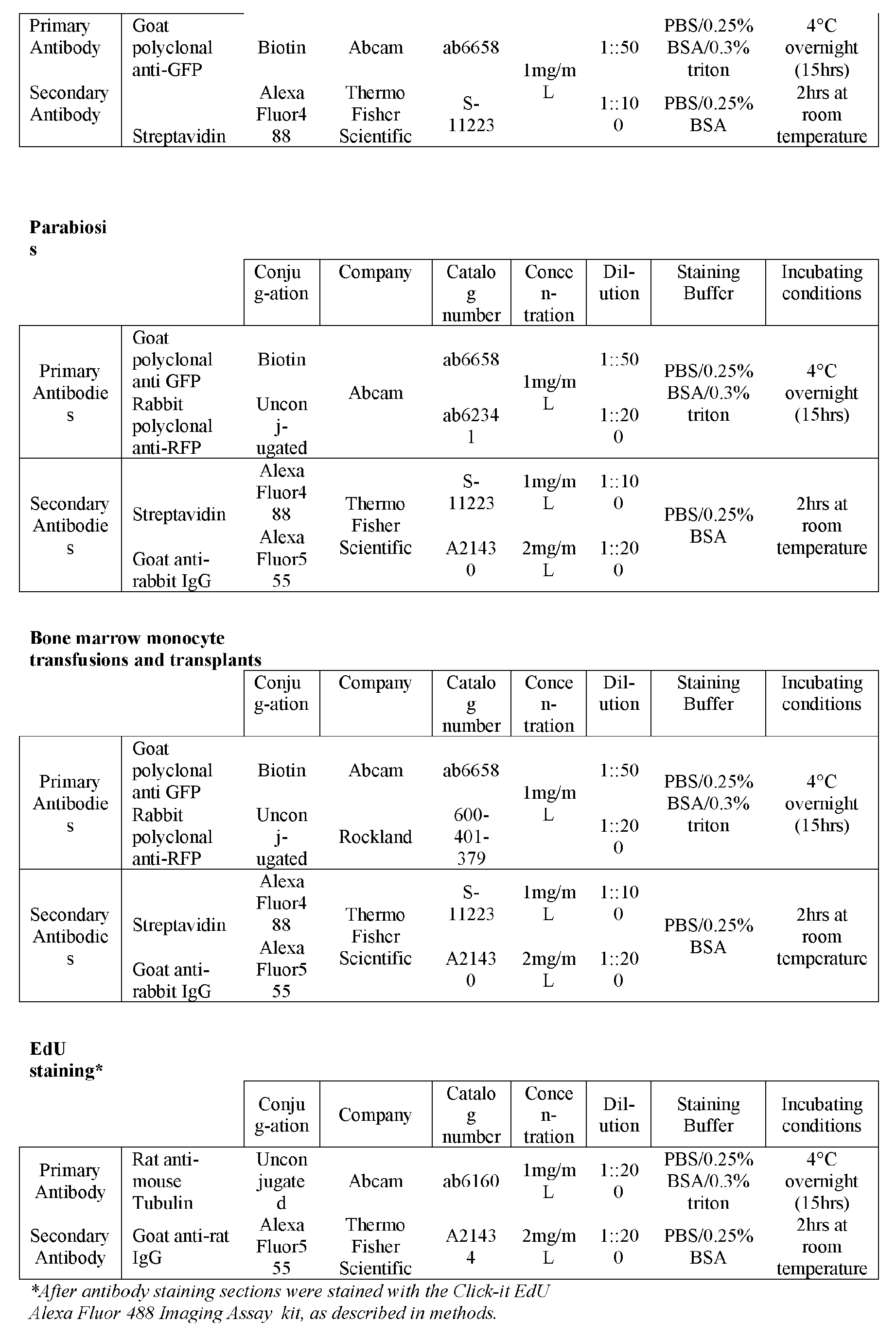

*Fluorescent TRAP staining.* Sections were prepared for fluorescent TRAP by incubating with TRAP incubation solution (112 mM sodium acetate, 76 mM sodium tartate, and 11 mM sodium nitrite, pH 4.1-4.3) at room temperature for 10 minutes. Buffer was removed and incubated with ELF97 substrate (Molecular Probes E6589, 2mM) at a concentration of 125µM in TRAP incubation solution for 15 min under UV light and washed 2 times with PBS for 5 minutes. Nuclear stain used was TO-PRO-3 Iodide (Fisher T3605) 1:4000 in PBS for 5 minutes. Mounting media was 75% glycerol in PBS. Images were acquired using an inverted Zeiss LSM880 laser scanning confocal microscope with Argonion 488nm, Diode 405-30nm, DPSS 561-10nm, HeNe 633nm laser lines and Plan-Apochromat 40X/1.4 N.A. DIC (UV) VIS-IR oil objective. Histological sections of 15µm thickness were tile-scanned at 1.5µm Z-stacks in ZEN black and processed using ZEN lite. Sections were analyzed using Imaris in 3D view and individual Z stacks to quantify TRAP⁺ multinuclear cells and YFP and Tomato labeling. Pictures of the region of interest were generated in tiff format and analyzed by ImageJ software using ROI manager to calculate the mean fluorescence intensity (MFI) of individual osteoclasts. Numerical values were plotted using GraphPad Prism.

*Animal imaging by computed tomography. NanoSPECT*/*CT:* Mice were anesthetized under Isofluorane anesthesia and placed on an imaging table containing an animal bed equipped with a nosecone for gas inhalation and body temperature stabilization. For 3 week old mice a mouse bed was used and for mice 8 weeks and older a rat bed was used. Whole-body imaging of mice was acquired using a NanoSPECT/CT scanner (Mediso) for non-invasive and longitudinal monitoring of the 3D skeletal structure. Each CT scan averaged 15 minutes and was acquired with an exposure time of 1,000ms and 240 projections set at a pitch of 1 degree. The tube energy of the X-ray was 55KVp and 145µA. The in-plane voxel size was medium generating a voxel size of 147µm³. Reconstructed images were analyzed using In Vivo Scope 2.0 (Bioscan, Inc.) software.

*microCT.* For tri-dimensional X-ray imaging by micro computed tomography, mice were sacrificed and bones placed in 70% ethanol until scanning. Bone microarchitecture was analyzed using the vivaCT40 (Scanco Medical, Switzerland). Entire femora or humeri were imaged at a resolution of 10.5µm (1 slice) with an X-ray energy of 70 kVp, 114 mA, and an integration time of 200ms. The machine was routinely calibrated using hydroxyapatite phantoms for density and geometry. Trabecular bone in femora or humeri from old mice was assessed in the metaphysis 20 slices below the growth plate using 150 slices. The trabecular region within the cortical bones (P21 mice) was determined in the femoral midshaft (100 slices up, 100 slices down). Pre-defined scripts from Scanco were used for the evaluation.

*Preparation of tissues and staining for Flow cytometry.* Yolk sac from E10.5 embryos was digested for 60 min at 37°C in PBS containing 5% FCS, Collagenase Type 4 (Worthington, final 4.2 U/ml) and DNAseI (Sigma-Aldrich, final 100 µg/ml). The digestion reaction was stopped by incubation with 12.5 mM EDTA. Fetal liver was gently dissociated between the frosted ends of glass slides, and was then digested for 30 minutes using the same digestion enzyme mix as yolk sac. Blood was collected from anesthetized mice by retro-orbital venous sinus bleeding or cardiac puncture using a 1mL syringe and a 26G needle rinsed with 100mM EDTA (sigma E4884). The collected blood was lysed with 3 mL of red blood cell lysis buffer (155mM NH₄Cl sigma A9434, 10mM NaHCO₃ sigma S5761 and 0.1mM EDTA sigma E4884) for 5 mins, washed with 10mL of FACS buffer (PBS, 0.5% BSA and 2mM EDTA) and centrifuged at 320g for 7 minutes at 4°C. All mice were perfused with 10ml of PBS after blood withdrawal. Bone marrow cells were harvested by crushing or flushing femurs and tibias using a syringe and a 26G needle with 10mL of PBS / 5% FCS or RPMI supplemented with 10% FBS. Bone marrow was dissociated by gently pipetting up and down with a 1mL or 10mL pipette. Spleens were gently dispersed between frosted slides and digested for 30 min at 37 °C in PBS with 5% FCS containing Collagenase Type 4 (Worthington) at a final concentration of 4.2 U/ml and 100 µg/ml DNAseI (Sigma-Aldrich). The reaction was stopped by incubation with 12.5 mM EDTA. Peritoneal cells were harvested by flushing the peritoneal cavity with 10mL PBS/5% FCS heated to 37°C. Adult brain and liver were dissected, cut into small fragments, and incubated at 37°C for 30 min in enzyme mix consisting of PBS with 1mg/ml collagenase D (Sigma, 11088882001), 100 U/ml DNase I (Sigma, DN25), 2.4mg/ml of dispase (Fisher, 17105-041) and 3% FBS (ThermoFisher Scientific 10438026) or PBS containing 4.2 U/ml Collagenase type 4 (Worthington), 100 µg/ml DNAseI, 2.4mg/ml of Dispase (Gibco) and 3% FCS at 37°C for 30 min. After enzyme digestion all tissues were further dissociated by mechanical disruption using 100µm cell strainers (Falcon, 352360) and a 3ml syringe plunger in 6 well plates containing 4mL or 5mL of cold FACS buffer or PBS / 5% FCS. Single cell suspensions were transferred to 5mL FACS tubes and pelleted by centrifugation at 320g for 7 minutes at 4°C. The cell pellets were resuspended in FACS buffer containing purified anti-mouse CD16/32 antibody (1:100, Biolegend 101301), 5% normal mouse (Fisher, 015-000-120), 5% normal rat (Fisher 012-000-120) and 5% normal rabbit serum (Fisher, 011-000-120) and incubated for 10 minutes on ice or directly stained using blocking antibodies in the staining mixtures. Samples were immunostained with fluorochrome-conjugated antibodies for 30 minutes on ice, and analyzed by flow cytometry using an LSRFortessa or an LSR II (BD-Bioscience). Full list of antibodies for flow cytometry is provided in **Table 3.** Each sample was stained with Hoechst (ThermoFisher Scientific, Hoechst 33258, 1µg/ml) or DAPI (Applichem, 1µg/25ml) moments prior to flow cytometry acquisition.

**Table 3.**

| **Antibody** | **Clone** | **Fluorochrom e** | **Company** | **Dilution** |
|---|---|---|---|---|
| CD3 | 145-2C11 | bio | eBioscience | 1/1000 (BM, Y, FL) |
| CD3 | 145-2C11 | BV711 | BD Pharmingen | 1/100 (BM, SP, LV, K, BR) |
| | | | | 1/400 (E) |
| CD3 | 145-2C11 | APC-Cy7 | BD Pharmingen | 1/100 (BL) |
| CD3 | 145-2C11 | PE | Biolegend | 1/100 (BM) |
| CD3 | 17A2 | eF450 | eBioscience | 1/200 (BL) |
| CD11b | M1/70 | bio | eBioscience | 1/1000 (BM, Y, FL) |
| CD11b | M1/70 | PE-Cy7 | eBioscience | 1/200 (BL, BM, SP, LV, K, BR, LN, E) |
| CD11b | M1/70 | BV711 | Biolegend | 1/200 (BM) |
| CD19 | 1D3 | bio | eBioscience | 1/500 (BM, Y, FL) |
| CD19 | 1D3 | BV711 | BD Pharmingen | 1/100 (BM, SP, LV, K, BR) |
| | | | | 1/200 (BL) |
| CD19 | 1D3 | PE-Cy7 | BD Pharmingen | 1/400 (BL) |
| B220 | RA3-6B2 | bio | eBioscience | 1/400 (BM,Y, FL) |
| Gr1 | RB6-8C5 | bio | eBioscience | 1/1000 (BM,Y, FL) |
| NK1.1 | PK136 | bio | eBioscience | 1/2000 (BM, Y, FL) |
| NKp46 | 29A1.4 | BV711 | BD Pharmingen | 1/100 (BM, SP, LV, K, BR) |
| NKp46 | 29A1.4 | AlexaFluor64 7 | BD Pharmingen | 1/200 (BL) |
| Ter119 | Ter119 | bio | eBioscience | 1/500 (BM, Y, FL) |
| Ter119 | Ter119 | BV711 | BD biosciences | 1/200 (BM) |
| CD11b | M1/70 | PE | BD Pharmingen | 1/1600 (BM) |
| CD11b | M1/70 | APC-eF780 | eBioscience | 1/600 (SP, Y, FL, LV, LN, BR, P) |
| CD11c | N418 | APC | eBioscience | 1/150 (SP, BM,YS FL, LV, LN, BR, P) |
| CD45 | 30-F11 | PE | eBioscience | 1/150 (SP, BM, Y, FL, LV, LN, BR, P) |
| CD45 | 30-F11 | APC-eF780 | eBioscience | 1/200 (Y, FL, P) |
| | | | | 1/50 (BM, SP, LV, K) |
| | | | | 1/100 (BR, LN, E) |
| CD45 | 30-F11 | eF450 | eBioscience | 1/50 (BM) |
| CD48 | HM48-1 | BV421 | BD Pharmingen | 1/400 (BM,Y, FL) |
| CD48 | HM48-1 | APC | Biolegend | 1/100 (BM) |
| CD115 | AFS98 | APC | eBioscience | 1/150 (SP, BM,Y, FL, LV, LN, BR, P) |
| CD115 | AFS98 | APC | Biolegend | 1/100 (BM) |
| CD115 | AFS98 | BV605 | Biolegend | 1/200 (BL, BM, SP, LV, K, BR) |
| CD115 | AFS98 | PE | eBioscience | 1/100 (SP, BM, LV, LN, BR, P) |
| CD117 | 2B8 | PE-Cy7 | eBioscience | 1/3000 (Y, FL) |
| CD117 | 2B8 | APC | eBioscience | 1/400 (BM,Y, FL) |
| CD117 | 2B8 | APC-Cy7 | Biolegend | 1/100 (BM) |
| CD117 | 2B8 | BV605 | BD biosciences | 1/100 (BM) |
| CD150 | TC15-12F1 | PE-Cy7 | BioLegend | 1/25 (Y, FL) |
| | | | | 1/100 (BM) |
| F4/80 | BM8 | PE-Cy5 | eBioscience | 1/300 (SP, Y, FL, LV, LN, BR, P) |
| F4/80 | BM8 | eF450 | eBioscience | 1/100 (BM, BR, LN, E) |
| Gr-1 | RB6-8C5 | PECy5.5 | eBioscience | 1/3000 (SP, BM, Y, FL, LV, LN, BR, P) |
| Gr-1 | RB6-8C5 | eF450 | eBioscience | 1/1000 (Y, FL) |
| Gr-1 | RB6-8C5 | BV711 | Biolegend | 1/100 (BM) |
| Ly6C | HK1.4 | PerCpCy5.5 | eBioscience | 1/800 (Y, FL) |
| Ly6C | HK1.4 | BV421 | Biolegend | 1/200 (BL) |
| | | | | 1/400 (SP, LV, K, BR) |
| Ly6C | HK1.4 | BV510 | Biolegend | 1/200 (BM) |
| Ly6C | HK1.4 | AlexaFluor48 8 | Biolegend | 1/50 (BM) |
| Ly6G | 1A8 | BV510 | Biolegend | 1/100 (BM) |
| | | | | 1/200 (Bl, LN) |
| | | | | 1/400 (SP, LV, K, BR) |
| Ly6G | 1A8 | BV711 | BD Pharmingen | 1/200 (BM) |
| MHCII | M5/114.15. 2 | Alexa Fluor 700 | eBioscience | 1/150 (SP, BM,Y, FL, LV, LN, BR, P) |
| MHCII | M5/114.15. 2 | AlexaFluor70 0 | Biolegend | 1/100 (BM) |
| | | | | 1/200 (BL, SP, LV, K, BR) |
| Seal | D7 | PercpCy5.5 | eBioscience | 1/500 (BM,Y, FL) |
| Sca-1 | D7 | BV421 | BD Pharmingen | 1/200 (BM) |
| Sca-1 | D7 | BV711 | Biolegend | 1/100 (BM) |
| Streptavidi n | | V500 | BD Biosciences | 1/800 (BM, Y, FL) |
| SiglecF | E50-2440 | AlexaFluor64 7 | BD Pharmingen | 1/100 (BM, LN) |

| | | | | |
|---|---|---|---|---|
| BM: Bone marrow; SP: Spleen; BL: Blood; Y: Yolk sac; FL: Fetal Liver; LV: Liver; LN: Lung; BR: Brain; P: PEC: E: Epidermis; K: Kidney. | | | | |

All analysis was conducted using FlowJo (Tree Star). In all tissues single live cells were gated by exclusion of dead cells labeled positive by Hoechst or DAPI, side scatter (SSC-A) and forward scatter (FSC-A) and doublet exclusion using forward scatter width (FSC-W) against (FSC-A), as previously described. In order to calculate cell numbers per organ or per gram of tissue, organs were weighted, cell suspensions were prepared from a weighted amount (20 to 500mg) of tissue, and the number of cells per gram of tissue was determined using a cell counter (GUAVA easyCyte HT).

*Statistical analysis and reproducibility.* Data are shown as mean with individual values per mouse represented as circles, unless stated otherwise. Statistical significance was analyzed with GraphPad Prism using unpaired t-tests and two-way ANOVA with Tukey's multiple comparisons test as indicated in the figure legends. Significance was considered at P value (P) *P < 0.05; **P < 0.01; ***P < 0.001; ****P < 0.0001. The n value represents biological replicates. Experiments were repeated to ensure reproducibility of the observations. Equal variance was assumed for cell-counting experiments. No statistical methods were used to predetermine sample size.

### Example 1: Osteoclast origins and maintenance

*In vitro,* osteoclasts arise by fusion of HSC-derived precursors and require expression of Csf1r and Tnfrsf11a (Rank). To probe the origin of osteoclasts *in vivo, Csf1r^{Cre}; Csf1r*^{*fl*/}*^{fl};* and *Csf1r^{Cre}; Tnfrsf11a*^{*fl*/*fl*} mice were generated. These mice presented with an osteopetrotic phenotype similar to *Csf1* (*op op*), *Csf1r*, and *Tnfrsf11a* mutants and characterized in young mice by lack of teeth eruption, skull and skeletal deformities with shortness of long bones, increased bone density, and lack of osteoclasts and hematopoietic cells **(****Figure 1A****;** **Figure 5B**). To confirm that osteoclast differentiation requires expression of Tnfrsf11a and Csf1r in HSC-derived precursors, *Flt3^{Cre}; Tnfrsf11a*^{*fl*/*fl*} mice, *Flt3^{Cre};Csf1r*^{*fl*/*fl*} mice, and *Vav^{Cre}; Csf1r*^{*fl*/*fl*}mice were generated. Surprisingly, all young mice had normal teeth (**Figure 1B**) and bone morphology (**Figure 6A-C**), normal bone marrow cellularity (**Figure 1C**), and normal osteoclast numbers (**Figure 1D**) in comparison to control littermates. However, *Flt3^{Cre}; Tnfrsf11a*^{*fl*/*fl*} mice, *Flt3cre;Csf1r*^{*fl*/*fl*} mice, and *Vav^{Cre}; Csf1r*^{*fl*/*fl*}mice lost their osteoclasts over time (**Figure 1D**), and by 22-60 weeks of age had increased trabecular bone density (**Figure 1E**), decreased hematopoietic cell numbers in the long bones (**Figure 1C****;** **Figure 6D-G**) and tri-dimensional X-ray imaging by micro computed tomography (microCT) confirmed increased bone mass while bone formation measured by calcein incorporation was similar to control(**Figure 1F****,** **Figure 7A-E**).

Mice lacking *Csf1r* expression in *Flt3^{Cre}*-expressing progenitors presented with a similar phenotype: *Flt3^{Cre}; Csf1r*^{*fl*/*fl*} mice have normal teeth and bone morphology at 4 week of age (**Figure 1B****,** **Figure 6C**), but osteoclast number are decreased over time and bone mass is increased at 22 weeks of age (**Figure 1D-E,** **Figure 7A-E**). In a complementary approach to delete *Csf1r* in HSC-derived progenitors, *Vav^{Cre};Csf1r*^{*fl*/*fl*} mice were generated. Again, *Vav^{Cre};Csf1r*^{*fl*/*fl*} mice were not osteopetrotic at birth, had normal teeth (**Figure 1B**), and hematopoietic cell numbers in long bones at 4 week of age (**Figure 1C**). However, they developed a late-onset osteopetrosis similar to *Flt3^{Cre}; Tnfrsf11a*^{*fl*/*fl*} and *Flt3^{Cre}; Csf1r*^{*fl*/*fl*} mice: at ~60 weeks of age *Vav^{Cre};Csf1r*^{*fl*/*fl*} mice had decreased osteoclasts and hematopoietic cell numbers in long bones (**Figure 1C-D**), enlarged trabecular bone (**Figure 1E**) and increased bone mass (**Figure 1F****,** **Figure 7A-E**). These data suggested that osteoclast development, tooth eruption and the development of bone and the bone marrow cavity requires precursors that express Csf1r, but not *Flt3^{Cre}* or *Vav^{Cre}* although postnatal contribution of *Flt3^{Cre}* / *Vav^{Cre}* expressing cells is important for optimal osteoclast function in adults and aging mice.

Fetal expression of *Csf1r^{Cre}* and *Flt3^{Cre}* was analyzed using a fluorescent reporter in *Csf1r^{Cre}; Rosa26^{LSL-YFP}* and *Flt3^{Cre}; Rosa26^{LSL-YFP}* mice (**Figure 1 G-I**). TRAP⁺ multinucleated cells that appear at embryonic day (E)15 in ossification centers are labeled with YFP in *Csf1r^{Cre};Rosa26^{LSL-YFP}* mice and osteoclasts remain YFP positive throughout life (**Figure 1** **G,I;** **Figure 8A-E**), but gain expression of YFP after birth in *Flt3^{Cre};Rosa26^{LSL-YFP}* mice despite colonization of the fetal bone marrow by *Flt3*^{*Cre*+} YFP⁺ hematopoietic cells (**Figure 1** **H;** **Figure 8A-E**). These data suggested that although postnatal contribution of HSC-derived cells is important for optimal osteoclast maintenance and function in adults and aging mice, osteoclast development, tooth eruption, the development of bone, and the bone marrow cavity requires precursors independent from the HSC lineage, possibly from the embryonic EMP lineage of resident macrophages because *Csf1r^{Cre}* mice allow deletion of target genes in both the embryonic EMP lineage and in the HSC lineage, while *Flt3^{Cre}* and *Vav^{Cre}* (**Figure 9A-D**) are not expressed in the EMP lineage.

In support of this hypothesis, it was found that TRAP⁺ multinucleated cells develop in ~E15 ossification centers from *Myb*-deficient embryo, which lack HSC but still support the development of EMP-derived macrophages (**Figure 2A****, B**). In addition, TRAP⁺ multinucleated cells are labeled with YFP in *Csf1r^{Mer-iCre-Mer}; Rosa26^{LSL-YFP}* mice pulsed at E8.5 with a single dose of hydroxytamoxifen (4-OHT), which labels EMP but not HSC (**Figure 2C****, D;** **Figure 8A-E**). Altogether, these results indicate that fetal osteoclasts arise from EMP in ossification centers.

Whether EMP are required for bone development was investigated. *Tnfrsf11a* is expressed by osteoclasts, but its expression is also a hallmark of EMP-derived pMacs that colonize the developing embryo. In two independent lines of *Tnftsf11a^{Cre}* knock-in mice, Cre-mediated expression of a *Rosa26^{LSL-YFP}* fluorescent reporter is achieved with high efficiency in fetal macrophages but with low efficiency or not at all in HSC and their progeny in blood and tissues (**Figure 9A-D**). It was therefore hypothesized that conditional deletion of *Csf1r* in *Tnfrsf11a^{Cre}* would recapitulate macrophage deficiency observed in *Csf1r*-deficient mice, while leaving the HSC lineage unaffected. To test osteoclast and bone development in this model, two independent lines of *Tnfrsf11a^{Cre};Csf1r*^{*fl*/*fl*} mice were generated (**Figure 2E-J**). *Tnfrsf11a^{Cre};Csf1r*^{*fl*/*fl*} lack tissue macrophages such as brain microglia and epidermal Langerhans cells at birth while development of HSCs and blood cells was preserved (**Figure 6A-N**). They present with a severe osteopetrotic phenotype including the lack of tooth eruption **(****Figure 2E****)**, misshaped skulls and short long bones (**Figure 2F****-H;** **Figure 5A-G)** with few osteoclasts (**Figure 2I**), increased bone density and initially lack a bone marrow cavity (**Figure 2F****;** **Figure 6A-N**). In contrast to *Csf1r*-deficient mice, however, osteoclasts and hematopoietic cells progressively colonized the long bones of *Tnfrsf11a^{Cre};Csf1r*^{*fl*/*fl*} mice during the first month of life (**Figure 21;** **Figure 5A****-G;** **Figure 6A-N**) although the mice remained toothless and skull and long bone deformity persisted (**Figure 2E****-H;** **Figure 5A-****G**). Calcein incorporation was similar in *Tnfrs11a^{Cre};Csf1r*^{*fl*/*fl*} mice and their littermate controls (**Figure 10A-F**). In a complementary approach ablation of *Csf1r* expression in E10.5 embryos using a single dose of tamoxifen in *Rosa26-creER*^{*T2*+}*;Csf1r*^{*fl*/*-*} mice resulted in defective tooth eruption in 3 out of 4 pups at 21 days of age (**Figure 9A-D**). Altogether, this analysis supports a model in which EMP-derived embryonic osteoclasts are needed for teeth eruption, normal skull shape and optimal formation of long bones, and the timely colonization of long bones by hematopoietic progenitors, while HSC-derived osteoclasts are important for the maintenance of bone mass after birth and later in life, although they may partially rescue bone development in the absence of EMP-derived osteoclasts in *Tnfrs11a^{Cre};Csf1r*^{*fl*/*fl*} mice.

### Example 2: Therapeutic approach for the treatment of osteopetrosis

To probe the mechanisms that underlie the contribution of HSC-derived blood cells to osteoclast maintenance as well as the lifespan and dynamics of osteoclasts *in vivo,* time-course parabiosis experiments were performed **(****Figure 3A****).** After 4 to 8 weeks of shared blood circulation between *Csf1r^{Cre};Rosa26^{LSL-YFP}* and *Csf1r^{Cre};Rosa26^{LSL-tdTomato}* parabionts, all osteoclasts, defined as TRAP⁺ multinucleated cells lining the bone surface, expressed both YFP and tdTomato within the same cells (**Figure 3A****, B**). No other cell was found to co-express YFP and tdTomato in bones (**Figure 3B**). This is compatible with the presence of nuclei from both partners in individual osteoclasts. Moreover, when parabionts were separated after 4 weeks (**Figure 3C**), most recipient YFP+ osteoclasts retained tdTomato staining 14 weeks after separation, and two third of osteoclasts from former parabionts still expressed both YFP and tdTomato 24 weeks after separation (**Figure 3C**). TdTomato signal intensity per YFP osteoclast increased during the 8-week period of shared blood circulation, and decreased after separation (**Figure 11A-C**). Most murine osteoclasts have ~5 (3 to 7) nuclei, with a modest increase of nuclei per cell between 1 month and 6 months of age (**Figure 3E**). These data therefore suggest that individual osteoclast syncytia are long lived, but acquire new nuclei one by one every 4 to 8 weeks from circulating blood cells so that it takes more than 6 months to renew all 5 nuclei from a given osteoclast.

The number and fusion-rate of HSC-derived nuclei acquired by osteoclasts in short-term 5-ethynyl-2'-deoxyuridine (EdU) incorporation studies was calculated. A single intravenous pulse of EdU (20µg/g) labels mitotic nuclei, is bioavailable in the bone marrow for ~90 min, and -50% of bone marrow and blood monocytic cells are EdU⁺ for ~48hrs (**Figure 11A-C**). It was observed that -1 to 2% of osteoclasts were labelled after 72 hrs, with only 1 EdU⁺ nucleus per osteoclast in 90% of positive cells (**Figure 3F**) suggesting acquisition by osteoclasts of single post-mitotic nuclei at a time. The proportion of osteoclasts acquiring a new nucleus can be estimated ~0.5 to 2 % per day in this model, compatible with individual nuclei being replaced every - 2 months. Altogether the studies above suggested a model (**Figure 3G**) where: osteoclasts that control skeletal development develop in ossification centers from EPM, while their post-natal maintenance is mediated via the serial acquisition by long-lived syncytia of new nuclei from HSC-derived blood leukocytes, rather than by *de novo* renewal by lateral fusion or proliferation of osteoclast precursors.

A prediction from this model is that osteopetrosis due to a recessive mutation affecting osteoclasts function may be rescued or prevented through parabiosis with a wild-type partner. Parabiosis experiments between 4 week-old cathepsin K deficient mice, which develop an adult-onset form of osteopetrosis known as pycnodysostosis, and cathepsin K^{+/-} or cathepsin K^{-/-} littermates and between wild type mice as control showed a reduction of bone volume in 10 weeks old cathepsin K^{-/-} mice paired with cathepsin K^{+/-} littermates (**Figure 4A**), suggesting that circulating blood cells carrying a wild type cathepsin K allele are sufficient to reduce bone density. To confirm that expression of a donor-derived gene by recipient osteoclasts results from fusion with blood circulating monocytic cells, intravenous injections of Kit⁻ Ly6C⁺ cells from the bone marrow of *Csf1r^{Cre};Rosa26^{LSL-tdTomato}* into *Csf1r^{Cre};Rosa26^{LSL-YFP}* recipients were performed (**Figure 4B****;** **Figure 12A-C**). This resulted in stable expression of tdTomato in 20-40% of osteoclasts 1 week and 8 weeks after transfusion, in the absence of other donor-derived blood cells or bone marrow progenitors (**Figure 4B****, C;** **Figure 12A-C**). These results suggest that parabiosis or an appropriate regimen of transfusion can achieve expression of a donor-derived gene by recipient osteoclasts in the absence of HSC chimerism, and in addition that this effect can last for several months.

Partial rescue of osteopetrosis occurs postnatally in *Tnfrsf11a^{Cre};Csf1r*^{*fl*/*fl*} mice, suggesting that transfusion of monocytic cells may also be able to rescue bone development in early-onset congenital osteopetrosis in the absence of a bone marrow transplantation. Intra-peritoneal injections of Kit⁻ Ly6C⁺ monocytic cells from *Csf1r^{Cre};Rosa26^{LSL-YFP}* into *Csf1r^{Cre}; Csf1r*^{*F*/*F*} neonates starting from post-natal day 5, resulted in complete or partial rescue of teeth eruption (**Figure 4D**) and long bone development as assessed by CT-Scan, with the development of a bone marrow cavity (**Figure 4D**) at day 14 in infant mice from 3 different litters (**Figure 4D****,** **Figure 13**). In all mice, histology of femurs indicated the presence of numerous YFP+ TRAP+ osteoclasts lining the bone (**Figure 4E****, F**). Histology and flow cytometry analyses showed the lack of YFP+ circulating blood cells or bone marrow progenitors **(****Figure 4E****, G**), indicating absence of HSC engraftment. These data suggest that transfusion of monocytic cells can rescue bone development in early-onset autosomal recessive osteopetrosis in infant mice, in the absence of HSC transplantation.

### Summary

The results described above show that osteoclasts originating from EMP are essential for normal bone development. Moreover, it is shown that osteoclasts are long-lived in adult and their function is maintained by iterative fusion of individual HSC-derived circulating cells with existing syncytia. In the absence or deficiency of EMP-derived osteoclasts, however, their timely replacement by transfusion with monocytic cells can rescue bone development in early-onset osteopetrotic mice in the absence of bone marrow transplantation. This could contribute to the treatment of early onset osteopetrosis, since the current treatment by bone marrow transplantation. This is of potential clinical relevance because bone marrow/HSC transplantation, the standard of care in early-onset osteopetrosis in mice and human, requires irradiation or chemotherapy which carry the risk of threatening infections and is frequently performed late in patients who already suffer severe complications and is plagued by a ~48% overall survival at 6-years. In addition, the original mechanism that mediates osteoclast maintenance suggests that they represent a unique target for gene transfer by cellular therapies based on transfusion of wild type or engineered monocytic cells to modulate osteoclast activity and bone remodeling in adults. Accordingly, these results demonstrate that monocytic cells may be effective in methods for treating osteopetrosis, and to modulate osteoclast activity and bone remodeling.

### Example 3: Ex vivo gene therapy for the treatment of a form of osteopetrosis: pycnodysostosis

This example demonstrates the prophetic use of monocytic cells obtained from a subject and engineered to express a gene, such as *CTSK*, for the treatment of osteopetrosis in the subject.

Pycnodysostosis is an autosomal recessive form of osteopetrosis, due to loss of function mutations in the cathepsin K gene (*CTSK*), compatible with life, and characterized by short stature, deformity of the skull maxilla and phalanges, increased density of the bones, osteosclerosis, and fragility of bone. There is no treatment for this disease as the relatively comparably milder symptoms do not qualify for bone marrow transplantation.

Subjects suspected of having or diagnosed as having cathepsin K deficiency will be selected for a gene therapy trial and will undergo GMP grade collection of peripheral blood monocytes by apheresis and/or elutriation. Purified monocytes will then be transduced with a lentivirus designed to express a cDNA coding for the wild type allele of *CTSK* under a strong promoter (*e.g.*, CMV). Transduced monocytes will then be washed to remove contaminating viral particles and rested for 12 hours followed by reinfusion to the patient. Assuming ~10⁹ monocytes can be collected, the number of cells transferred per kilogram of body weight would be >10⁷. Following the auto-transfusion, the subject will be monitored at regular intervals for clinical and radiological signs of pycnodysostosis in order to determine the efficiency of the procedure and the frequency with which it should be repeated.

It is predicted that subjects suspected of having or diagnosed as having pycnodysostosis and receiving therapeutically effective amounts of monocytic cells engineered to express *CTSK* will display reduced severity or elimination of one or more symptoms associated with pycnodysostosis. Accordingly, these results will show that transfusion of monocytic cells engineered to express the wild-type version of one or more genes implicated in osteopetrosis is useful in methods for treating subjects in need thereof for the treatment of osteopetrosis.

### Example 4: Ex vivo gene therapy for the treatment of a form of osteopetrosis: severe infantile autosomal recessive osteopetrosis

This example demonstrates the prophetic use of monocytic cells obtained from a subject and engineered to express a gene, such as *TCIRG1, TNFRSF11a, CA2, CLCN7, OSTM1,* and/or *PLEKHM1,* for the treatment of osteopetrosis in the subject.

Infants diagnosed with severe infantile autosomal recessive osteopetrosis with defects in *TCIRG1, TNFRSF11a, CA2, CLCN7, OSTM1,* and/or *PLEKHM1* will be eligible for an early auto-transfusion of corrected monocytes (as described in Example 3), to try to prevent early developmental complications, including blindness and skeletal deformities, and increase the success of bone marrow transplantation, by restoring a bone marrow niche. The protocol will be carried out in a manner similar to that of Example 3, and monocytes will be transduced with a lentivirus designed to express a cDNA coding for the wild type allele of the deficient gene under a strong promoter, with a similar number of transferred cells per kg of weight. Following the auto-transfusion, subjects will be monitored at regular intervals for clinical and radiological signs, and possibly bone biopsy in order to determine the efficiency of the procedure. In addition, collection of monocytes and hematopoietic stem cells/progenitors at the same time would allow to proceed with the auto transfusion of transduced monocytes, while stem cells would undergo gene editing in view of auto transplantation and long term genetic rescue.

It is predicted that subjects suspected of having or diagnosed as having severe infantile autosomal recessive osteopetrosis and receiving therapeutically effective amounts of monocytic cells engineered to express *TCIRG1, TNFRSF11a, CA2, CLCN7, OSTM1,* and/or *PLEKHM1* will display reduced severity or elimination of one or more symptoms associated with severe infantile autosomal recessive osteopetrosis. Accordingly, these results will show that transfusion of monocytic cells engineered to express the wild-type version of one or more genes implicated in osteopetrosis is useful in methods for treating subjects in need thereof for the treatment of osteopetrosis.

### REFERENCES

1. Coccia, P. F. et al. Successful bone-marrow transplantation for infantile malignant osteopetrosis. The New England journal of medicine 302, 701-708, doi:10.1056/NEJM198003273021301 (1980).
2. Sorell, M. et al. Marrow transplantation for juvenile osteopetrosis. The American journal of medicine 70, 1280-1287 (1981).
3. Lacey, D. L. et al. Osteoprotegerin ligand is a cytokine that regulates osteoclast differentiation and activation. Cell 93, 165-176 (1998).
4. Wiktor-Jedrzejczak, W. et al. Total absence of colony-stimulating factor 1 in the macrophage-deficient osteopetrotic (op/op) mouse. Proceedings of the National Academy of Sciences 87, 4828-4832, doi:10.1073/pnas.87.12.4828 (1990).
5. Kong, Y. Y. et al. OPGL is a key regulator of osteoclastogenesis, lymphocyte development and lymph-node organogenesis. Nature 397, 315-323, doi:10.1038/16852 (1999).
6. Dougall, W. C. et al. RANK is essential for osteoclast and lymph node development. Genes & development 13, 2412-2424 (1999).
7. Hsu, H. et al. Tumor necrosis factor receptor family member RANK mediates osteoclast differentiation and activation induced by osteoprotegerin ligand. Proceedings of the National Academy of Sciences of the United States of America 96, 3540-3545 (1999).
8. Yoshida, H. et al. The murine mutation osteopetrosis is in the coding region of the macrophage colony stimulating factor gene. Nature 345, 442-444 (1990).
9. Dai, X.-M. et al. Targeted disruption of the mouse colony-stimulating factor 1 receptor gene results in osteopetrosis, mononuclear phagocyte deficiency, increased primitive progenitor cell frequencies, and reproductive defects. Blood 99, 111-120, doi:10.1182/blood.V99.1.111 (2002).
10. Luchin, A. et al. Genetic and physical interactions between Microphthalmia transcription factor and PU.1 are necessary for osteoclast gene expression and differentiation. The Journal of biological chemistry 276, 36703-36710, doi:10.1074/jbc.M106418200 (2001).
11. Grigoriadis, A. E. et al. c-Fos: a key regulator of osteoclast-macrophage lineage determination and bone remodeling. Science 266, 443-448 (1994).
12. Lomaga, M. A. et al. TRAF6 deficiency results in osteopetrosis and defective interleukin-1, CD40, and LPS signaling. Genes & development 13, 1015-1024 (1999).
13. Tondravi, M. M. et al. Osteopetrosis in mice lacking haematopoietic transcription factor PU.1. Nature 386, 81-84, doi:10.1038/386081a0 (1997).
14. Takayanagi, H. et al. Induction and activation of the transcription factor NFATc1 (NFAT2) integrate RANKL signaling in terminal differentiation of osteoclasts. Developmental cell 3, 889-901 (2002).
15. Lau, R. Y. & Guo, X. A review on current osteoporosis research: with special focus on disuse bone loss. J Osteoporos 2011, 293808, doi:10.4061/2011/293808 (2011).
16. Walker, D. G. Bone resorption restored in osteopetrotic mice by transplants of normal bone marrow and spleen cells. Science 190, 784-785 (1975).
17. Walker, D. G. Control of bone resorption by hematopoietic tissue. The induction and reversal of congenital osteopetrosis in mice through use of bone marrow and splenic transplants. The Journal of experimental medicine 142, 651-663 (1975).
18. Ballet, J. J., Griscelli, C., Coutris, C., Milhaud, G. & Maroteaux, P. Bone-marrow transplantation in osteopetrosis. Lancet 2, 1137 (1977).
19. Nisbet, N. W., Menage, J. & Loutit, J. F. Bone-marrow transplantation in osteopetrosis. Lancet 2, 1236 (1977).
20. Ash, P., Loutit, J. F. & Townsend, K. M. Osteoclasts derived from haematopoietic stem cells. Nature 283, 669-670 (1980).
21. Orchard, P. J. et al. Hematopoietic stem cell transplantation for infantile osteopetrosis. Blood 126, 270-276, doi:10.1182/blood-2015-01-625541 (2015).
22. Xiao, Y. et al. Identification of the Common Origins of Osteoclasts, Macrophages, and Dendritic Cells in Human Hematopoiesis. Stem cell reports 4, 984-994, doi:10.1016/j.stemcr.2015.04.012 (2015).
23. Shalhoub, V. et al. Characterization of osteoclast precursors in human blood. British journal of haematology 111, 501-512 (2000).
24. Stadtfeld, M. & Graf, T. Assessing the role of hematopoietic plasticity for endothelial and hepatocyte development by non-invasive lineage tracing. Development 132, 203-213, doi:10.1242/dev.01558 (2005).
25. Taniguchi, N. et al. Stage-specific secretion of HMGB1 in cartilage regulates endochondral ossification. Molecular and cellular biology 27, 5650-5663, doi:10.1128/MCB.00130-07 (2007).
26. Schulz, C. et al. A lineage of myeloid cells independent of Myb and hematopoietic stem cells. Science 336, 86-90, doi:10.1126/science.1219179 (2012).
27. Gomez Perdiguero, E. et al. Tissue-resident macrophages originate from yolk-sac-derived erythro-myeloid progenitors. Nature 518, 547-551, doi:10.1038/nature13989 (2015).
28. Mass, E. et al. Specification of tissue-resident macrophages during organogenesis. Science 353, doi:10.1126/science.aaf4238 (2016).
29. Deng, L. et al. A novel mouse model of inflammatory bowel disease links mammalian target of rapamycin-dependent hyperproliferation of colonic epithelium to inflammation-associated tumorigenesis. Am J Pathol 176, 952-967, doi: 10.23 5 3 /aj path. 2010.090622 (2010).
30. Qian, B. Z. et al. CCL2 recruits inflammatory monocytes to facilitate breast-tumour metastasis. Nature 475, 222-225, doi:10.1038/nature10138 (2011).
31. Li, J., Chen, K., Zhu, L. & Pollard, J. W. Conditional deletion of the colony stimulating factor-1 receptor (c-fms proto-oncogene) in mice. Genesis 44, 328-335, doi:10.1002/dvg.20219 (2006).
32. Dai, X. M. et al. Targeted disruption of the mouse colony-stimulating factor 1 receptor gene results in osteopetrosis, mononuclear phagocyte deficiency, increased primitive progenitor cell frequencies, and reproductive defects. Blood 99, 111-120 (2002).
33. Benz, C., Martins, V. C., Radtke, F. & Bleul, C. C. The stream of precursors that colonizes the thymus proceeds selectively through the early T lineage precursor stage of T cell development. The Journal of experimental medicine 205, 1187-1199, doi:10.1084/jem.20072168 (2008).
34. Mucenski, M. L. et al. A functional c-myb gene is required for normal murine fetal hepatic hematopoiesis. Cell 65, 677-689 (1991).
35. Stadtfeld, M. & Graf, T. Assessing the role of hematopoietic plasticity for endothelial and hepatocyte development by non-invasive lineage tracing. Development 132, 203-213, doi:10.1242/dev.01558 (2005).
36. Hanada, R. et al. Central control of fever and female body temperature by RANKL/RANK. Nature 462, 505-509, doi:10.1038/nature08596 (2009).
37. Maeda, K. et al. Wnt5a-Ror2 signaling between osteoblast-lineage cells and osteoclast precursors enhances osteoclastogenesis. Nature medicine 18, 405-412, doi:10.1038/nm.2653 (2012).
38. Hameyer, D. et al. Toxicity of ligand-dependent Cre recombinases and generation of a conditional Cre deleter mouse allowing mosaic recombination in peripheral tissues. Physiol Genomics 31, 32-41, doi:10.1152/physiolgenomics.00019.2007 (2007).
39. Srinivas, S. et al. Cre reporter strains produced by targeted insertion of EYFP and ECFP into the ROSA26 locus. BMC developmental biology 1, 4 (2001).
40. Madisen, L. et al. A robust and high-throughput Cre reporting and characterization system for the whole mouse brain. Nat Neurosci 13, 133-140, doi:10.1038/nn.2467 (2010).
41. Schulz, C. et al. A lineage of myeloid cells independent of Myb and hematopoietic stem cells. Science 336, 86-90, doi:10.1126/science.1219179 (2012).
42. Gomez Perdiguero, E. et al. Tissue-resident macrophages originate from yolk-sac-derived erythro-myeloid progenitors. Nature 518, 547-551, doi:10. 10 38/nature13989 (2015).
43. Kamran, P. et al. Parabiosis in mice: a detailed protocol. J Vis Exp, doi:10.3791/50556 (2013).
44. Jiang, X. et al. Histological analysis of GFP expression in murine bone. J Histochem Cytochem 53, 593-602, doi:10.1369/jhc.4A6401.2005 (2005).
45. Filgueira, L. Fluorescence-based staining for tartrate-resistant acidic phosphatase (TRAP) in osteoclasts combined with other fluorescent dyes and protocols. J Histochem Cytochem 52, 411-414, doi:10.1177/002215540405200312 (2004).
46. Mass, E. et al. Specification of tissue-resident macrophages during organogenesis. Science 353, doi:10.1126/science.aaf4238 (2016).
47. Arndt, K. et al. SETD1A protects HSCs from activation-induced functional decline in vivo. Blood 131, 1311-1324, doi:10.1182/blood-2017-09-806844 (2018).
48. Grinenko, T. et al. Clonal expansion capacity defines two consecutive developmental stages of long-term hematopoietic stem cells. The Journal of experimental medicine 211, 209-215, doi:10.1084/jem.20131115 (2014).

## Claims

1. A composition comprising an effective amount of monocytic cells that have been obtained from a healthy donor for use in treating or preventing osteopetrosis in a subject in need thereof.

2. The composition for the use of claim 1, wherein the subject is **characterized by** decreased expression of one or more of *CA2, CLCN7, CTSK, CSF1R, IKBKG, ITGB3, OSTM1, PLEKHM1, TCIRG1, TNFRSF11A,* and *TNFSF11,* as compared to the monocytic cells of the donor.

3. The composition for the use of claim 1, wherein the osteopetrosis comprises one or more of stunted growth, skeletal deformity, increased likelihood of bone fracture, anemia, recurrent infections, hepatosplenomegaly, facial paralysis, abnormal cortical bone morphology, abnormal form of vertebral bodies, abnormal temperature regulation, abnormality of the ribs, abnormality of vertebral epiphysis morphology, bone pain, cranial nerve paralysis, craniosynostosis, hearing impairment, and hypocalcemia.

4. The composition for the use of claim 1, wherein the composition is formulated for intravenous administration by injection, infusion, or transfusion.

5. The composition for the use of claim 1, wherein the subject is a mammal, optionally wherein the mammalian subject is a human.

6. A composition comprising an effective amount monocytic cells engineered to express one or more genes selected from *CA2, CLCN7, CTSK, CSF1R, IKBKG, ITGB3, OSTM1, PLEKHM1, TCIRG1, TNFRSF11A,* and *TNFSF11,* for use in treating or preventing osteopetrosis in a subject in need thereof.

7. The composition for the use of claim 6, wherein the osteopetrosis comprises one or more of stunted growth, skeletal deformity, increased likelihood of bone fracture, anemia, recurrent infections, hepatosplenomegaly, facial paralysis, abnormal cortical bone morphology, abnormal form of vertebral bodies, abnormal temperature regulation, abnormality of the ribs, abnormality of vertebral epiphysis morphology, bone pain, cranial nerve paralysis, craniosynostosis, hearing impairment, and hypocalcemia.

8. The composition for the use of claim 6, wherein the composition is formulated for intravenous administration by injection, infusion, or transfusion.

9. The composition for the use of claim 6, wherein the subject is a mammal, and optionally wherein the mammalian subject is a human; or
wherein the subject is **characterized by** a cathepsin K deficiency and wherein the monocytic cells are engineered to express CTSK; or
wherein the subject is diagnosed as having pycnodysostosis and wherein the monocytic cells are engineered to express one or more genes selected from the group consisting of TCIRG1, TNFRSF11a, CA2, CLCN7, OSTM1, and PLEKHM1; or
wherein the subject is diagnosed as having severe infantile autosomal recessive osteopetrosis.

10. The composition for the use of claim 6, wherein the monocytic cells are obtained from the subject.

11. A donor monocytic cell line engineered to express one or more genes selected from *CA2, CLCN7, CTSK, CSF1R, IKBKG, ITGB3, OSTM1, PLEKHM1, TCIRG1, TNFRSF11A,* and *TNFSF11,* wherein the one or more genes is operably linked to a heterologous nucleic acid to form a chimeric nucleic acid construct.

12. The donor monocytic cell line of claim 11, wherein the heterologous nucleic acid encodes a selectable marker, optionally wherein the selectable marker is a bioluminescent protein, a fluorescent protein, a chemiluminescent protein, a xanthine-guanine phosphoribosyl transferase gene (gpt), or any combination thereof.

13. The donor monocytic cell line of claim 11, wherein the heterologous nucleic acid encodes one or more control sequences suitable for directing expression of the one or more genes in a monocytic cell, optionally wherein the one or more control sequences comprises a promoter.

14. The donor monocytic cell line of claim 11, wherein the cells comprise a vector encoding one or more genes selected from *CA2, CLCN7, CTSK, CSF1R, IKBKG, ITGB3, OSTM1, PLEKHM1, TCIRG1, TNFRSF11A,* and *TNFSF11.*

15. The donor monocytic cell line of claim 14, wherein the vector is a mammalian expression vector, a lentiviral vector, or transposon vector.

## Patentansprüche

1. Zusammensetzung, umfassend eine wirksame Menge an monozytären Zellen, die von einem gesunden Spender erhalten wurden, zur Verwendung bei der Behandlung oder Vorbeugung von Osteopetrose bei einem Subjekt, das diese benötigt.

2. Zusammensetzung zur Verwendung nach Anspruch 1, wobei das Subjekt durch eine verminderte Expression von einem oder mehreren von *CA2, CLCN7, CTSK, CSF1R, IKBKG, ITGB3, OSTM1, PLEKHM1, TCIRG1, TNFRSF11A* und *TNFSF11* im Vergleich zu den monozytären Zellen des Spenders charakterisiert ist.

3. Zusammensetzung zur Verwendung nach Anspruch 1, wobei die Osteopetrose eines oder mehrere von verkümmertem Wachstum, Skelettdeformität, erhöhter Wahrscheinlichkeit von Knochenbrüchen, Anämie, wiederkehrenden Infektionen, Hepatosplenomegalie, Gesichtslähmung, abnormer Morphologie der Rindenknochen, abnormer Form der Wirbelkörper, abnormer Temperaturregulierung, abnormer Morphologie der Wirbelepiphyse, Knochenschmerzen, Hirnnervenlähmung, Kraniosynostose, Hörstörungen und Hypokalzämie.

4. Zusammensetzung zur Verwendung nach Anspruch 1, wobei die Zusammensetzung zur intravenösen Verabreichung durch Injektion, Infusion oder Transfusion formuliert ist.

5. Zusammensetzung zur Verwendung nach Anspruch 1, wobei das Subjekt ein Säugetier ist, optional, wobei das Säugetier-Subjekt ein Mensch ist.

6. Zusammensetzung, umfassend eine wirksame Menge monozytärer Zellen, die konstruiert wurden, um ein oder mehrere Gene zu exprimieren, die aus *CA2, CLCN7, CTSK, CSF1R, IKBKG, ITGB3, OSTM1, PLEKHM1, TCIRG1, TNFRSF11A* und *TNFSF11*,ausgewählt sind, zur Verwendung bei der Behandlung oder Vorbeugung von Osteopetrose bei einem Subjekt, das diese benötigt.

7. Zusammensetzung zur Verwendung nach Anspruch 6, wobei die Osteopetrose eines oder mehrere von verkümmertem Wachstum, Skelettdeformität, erhöhter Wahrscheinlichkeit von Knochenbrüchen, Anämie, wiederkehrenden Infektionen, Hepatosplenomegalie, Gesichtslähmung, abnormer Morphologie der Rindenknochen, abnormer Form der Wirbelkörper, abnormer Temperaturregulierung, abnormer Morphologie der Wirbelepiphyse, Knochenschmerzen, Hirnnervenlähmung, Kraniosynostose, Hörstörungen und Hypokalzämie.

8. Zusammensetzung zur Verwendung nach Anspruch 6, wobei die Zusammensetzung zur intravenösen Verabreichung durch Injektion, Infusion oder Transfusion formuliert ist.

9. Zusammensetzung zur Verwendung nach Anspruch 6, wobei das Subjekt ein Säugetier ist und optional, wobei das Säugetier-Subjekt ein Mensch ist; oder
wobei das Subjekt durch einen Cathepsin K-Mangel charakterisiert ist und wobei die monozytären Zellen konstruiert wurden, um CTSK zu exprimieren; oder
wobei bei dem Subjekt Pyknodysostose diagnostiziert wird und wobei die monozytären Zellen konstruiert wurden, um ein oder mehrere Gene zu exprimieren, die aus TCIRG1, TNFRSF11a, CA2, CLCN7, OSTM1 und PLEKHM1 ausgewählt sind; oder
wobei bei dem Subjekt eine schwere infantile autosomal rezessive Osteopetrose diagnostiziert wird.

10. Zusammensetzung zur Verwendung nach Anspruch 6, wobei die monozytären Zellen von dem Subjekt erhalten werden.

11. Monozytäre Zelllinie eines Spenders, die konstruiert wurde, um ein oder mehrere Gene zu exprimieren, die aus *CA2, CLCN7, CTSK, CSF1R, IKBKG, ITGB3, OSTM1, PLEKHM1, TCIRG1, TNFRSF11A* und *TNFSF11* ausgewählt sind, wobei das eine oder die mehreren Gene funktionsfähig an eine heterologe Nukleinsäure verbunden sind, um ein chimäres Nukleinsäurekonstrukt zu bilden.

12. Monozytäre Zelllinie eines Spenders nach Anspruch 11, wobei die heterologe Nukleinsäure für einen selektierbaren Marker codiert, optional wobei der selektierbare Marker ein biolumineszentes Protein, ein fluoreszierendes Protein, ein chemilumineszentes Protein, ein Xanthin-Guanin-Phosphoribosyl-Transferase-Gen (gpt) oder eine beliebige Kombination davon ist.

13. Monozytäre Zelllinie eines Spenders nach Anspruch 11, wobei die heterologe Nukleinsäure für eine oder mehrere Kontrollsequenzen codiert, die geeignet sind, die Expression des einen oder der mehreren Gene in einer monozytären Zelle zu richten, wobei die eine oder mehreren Kontrollsequenzen optional einen Promoter umfassen.

14. Monozytäre Zelllinie eines Spenders nach Anspruch 11, wobei die Zellen einen Vektor umfassen, der ein oder mehrere Gene codiert, die aus *CA2, CLCN7, CTSK, CSF1R, IKBKG, ITGB3, OSTM1, PLEKHM1, TCIRG1, TNFRSF11A* und *TNFSF11* ausgewählt sind.

15. Monozytäre Zelllinie eines Spenders nach Anspruch 14, wobei der Vektor ein Säugetier-Expressionsvektor, ein lentiviraler Vektor oder ein Transposon-Vektor ist.

## Revendications

1. Composition comprenant une quantité efficace de cellules monocytaires qui ont été obtenues auprès d'un donneur sain destinée à être utilisée dans le traitement ou la prévention de l'ostéopétrose chez un sujet en ayant besoin.

2. Composition destinée à être utilisée selon la revendication 1, dans laquelle le sujet est **caractérisé par** une expression diminuée d'un ou plusieurs parmi *CA2, CLCN7, CTSK, CSF1R, IKBKG, ITGB3, OSTM1, PLEKHM1, TCIRG1, TNFRSF11A et TNFSF11,* par rapport aux cellules monocytaires du donneur.

3. Composition destinée à être utilisée selon la revendication 1, dans laquelle l'ostéopétrose comprend un ou plusieurs parmi un retard de croissance, une déformation du squelette, une probabilité accrue de fracture osseuse, une anémie, des infections récurrentes, une hépatosplénomégalie, une paralysie faciale, une morphologie osseuse corticale anormale, une forme anormale des corps vertébraux, une régulation anormale de la température, une anomalie des côtes, une anomalie de la morphologie de l'épiphyse vertébrale, des douleurs osseuses, une paralysie des nerfs crâniens, une craniosténose, une déficience auditive et une hypocalcémie.

4. Composition destinée à être utilisée selon la revendication 1, dans laquelle la composition est formulée pour une administration intraveineuse par injection, perfusion ou transfusion.

5. Composition destinée à être utilisée selon la revendication 1, dans laquelle le sujet est un mammifère, éventuellement dans laquelle le sujet mammifère est un humain.

6. Composition comprenant une quantité efficace de cellules monocytaires conçues pour exprimer un ou plusieurs gènes choisis parmi *CA2, CLCN7, CTSK, CSF1R, IKBKG, ITGB3, OSTM1, PLEKHM1, TCIRG1, TNFRSF11A et TNFSF11,* destinée à être utilisée dans le traitement ou la prévention de l'ostéopétrose chez un sujet en ayant besoin.

7. Composition destinée à être utilisée selon la revendication 6, dans laquelle l'ostéopétrose comprend un ou plusieurs parmi un retard de croissance, une déformation du squelette, une probabilité accrue de fracture osseuse, une anémie, des infections récurrentes, une hépatosplénomégalie, une paralysie faciale, une morphologie osseuse corticale anormale, une forme anormale des corps vertébraux, une régulation anormale de la température, une anomalie des côtes, une anomalie de la morphologie de l'épiphyse vertébrale, des douleurs osseuses, une paralysie des nerfs crâniens, une craniosténose, une déficience auditive et une hypocalcémie.

8. Composition destinée à être utilisée selon la revendication 6, dans laquelle la composition est formulée pour une administration intraveineuse par injection, perfusion ou transfusion.

9. Composition destinée à être utilisée selon la revendication 6, dans laquelle le sujet est un mammifère, et éventuellement dans laquelle le sujet mammifère est un humain ; ou
dans laquelle le sujet est **caractérisé par** un déficit en cathepsine K et dans laquelle les cellules monocytaires sont conçues pour exprimer le CTSK ; ou dans laquelle le sujet est diagnostiqué comme souffrant de pycnodysostose et dans laquelle les cellules monocytaires sont conçues pour exprimer un ou plusieurs gènes choisis dans le groupe constitué de TCIRG1, TNFRSF11a, CA2, CLCN7, OSTM1 et PLEKHM1 ; ou
dans laquelle le sujet est diagnostiqué comme souffrant d'ostéopétrose autosomique récessive infantile sévère.

10. Composition destinée à être utilisée selon la revendication 6, dans laquelle les cellules monocytaires sont obtenues auprès du sujet.

11. Lignée de cellules monocytaires donneuses conçue pour exprimer un ou plusieurs gènes choisis parmi CA2, *CLCN7, CTSK, CSF1R, IKBKG, ITGB3, OSTM1, PLEKHM1, TCIRG1, TNFRSF11A et TNFSF11,* dans laquelle l'un ou les plusieurs gènes sont liés de manière fonctionnelle à un acide nucléique hétérologue pour former une construction d'acide nucléique chimérique.

12. Lignée de cellules monocytaires donneuses selon la revendication 11, dans laquelle l'acide nucléique hétérologue code pour un marqueur sélectionnable, éventuellement dans laquelle le marqueur sélectionnable est une protéine bioluminescente, une protéine fluorescente, une protéine chimioluminescente, un gène de xanthine-guanine phosphoribosyl transférase (gpt), ou toute combinaison de ceux-ci.

13. Lignée de cellules monocytaires donneuses selon la revendication 11, dans laquelle l'acide nucléique hétérologue code pour une ou plusieurs séquences de contrôle appropriées pour diriger l'expression de l'un ou des plusieurs gènes dans une cellule monocytaire, éventuellement dans laquelle l'une ou les plusieurs séquences de contrôle comprennent un promoteur.

14. Lignée de cellules monocytaires donneuses selon la revendication 11, dans laquelle les cellules comprennent un vecteur codant pour un ou plusieurs gènes choisis parmi *CA2, CLCN7, CTSK, CSF1R, IKBKG, ITGB3, OSTM1, PLEKHM1, TCIRG1, TNFRSF11A et TNFSF11.*

15. Lignée de cellules monocytaires donneuses selon la revendication 14, dans laquelle le vecteur est un vecteur d'expression de mammifère, un vecteur lentiviral ou un vecteur transposon.
